(19) Europäisches Patentamt
European Patent Office
Office européen des brevets

(11) EP 3 780 226 B1

(12) **EUROPEAN PATENT SPECIFICATION**

(45) Date of publication and mention of the grant of the patent:
**11.05.2022 Bulletin 2022/19**

(21) Application number: **19777777.4**

(22) Date of filing: **28.03.2019**

(51) International Patent Classification (IPC):
***H01M 10/0567*** *(2010.01)* ***H01M 10/0568*** *(2010.01)*
***H01M 10/0525*** *(2010.01)* ***H01M 4/36*** *(2006.01)*
***H01M 4/38*** *(2006.01)* ***H01M 4/48*** *(2010.01)*
***H01M 4/525*** *(2010.01)* ***H01M 4/587*** *(2010.01)*
***H01G 11/06*** *(2013.01)* ***H01G 11/50*** *(2013.01)*
***H01G 11/64*** *(2013.01)* *H01G 11/46 (2013.01)*

(52) Cooperative Patent Classification (CPC):
**H01M 10/0525; H01G 11/06; H01G 11/50; H01G 11/64; H01M 4/364; H01M 4/386; H01M 4/483; H01M 4/525; H01M 4/587; H01M 10/0567; H01M 10/0568;** H01G 11/46; H01M 2220/30; H01M 2300/0025; Y02E 60/10

(86) International application number:
**PCT/JP2019/013815**

(87) International publication number:
**WO 2019/189670 (03.10.2019 Gazette 2019/40)**

(54) **NONAQUEOUS ELECTROLYTE SOLUTION AND NONAQUEOUS ELECTROLYTE BATTERY**
WASSERFREIE ELEKTROLYTLÖSUNG UND BATTERIE MIT WASSERFREIEM ELEKTROLYT
SOLUTION ÉLECTROLYTIQUE NON AQUEUSE ET BATTERIE À ÉLECTROLYTE NON AQUEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2018 JP 2018064085**

(43) Date of publication of application:
**17.02.2021 Bulletin 2021/07**

(73) Proprietors:
- **Mitsubishi Chemical Corporation**
  **Chiyoda-ku**
  **Tokyo 100-8251 (JP)**
- **MU Ionic Solutions Corporation**
  **Tokyo 100-8251 (JP)**

(72) Inventor: **NAKAZAWA, Eiji**
**Tokyo 100-8251 (JP)**

(74) Representative: **Vossius & Partner**
**Patentanwälte Rechtsanwälte mbB**
**Siebertstrasse 3**
**81675 München (DE)**

(56) References cited:
**EP-A1- 3 051 618**
**WO-A1-2008/050599**
**WO-A1-2013/146819**
**JP-A- 2012 178 339**
**JP-A- 2016 054 143**
**JP-A- 2016 143 449**
**JP-A- 2017 016 752**
**JP-B2- 4 033 074**
**US-A1- 2004 043 300**
**US-A1- 2015 364 794**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

The present invention relates to a nonaqueous electrolyte solution and a nonaqueous electrolyte battery, more particularly a nonaqueous electrolyte solution containing a specific compound in a specific amount, and a nonaqueous electrolyte battery including the nonaqueous electrolyte solution.

BACKGROUND ART

In recent years, nonaqueous electrolyte batteries such as lithium secondary batteries have been practically used in the applications such as vehicle-mounted power sources for driving electric vehicles and the like.

As means for improving the characteristics of a nonaqueous electrolyte battery, numerous studies have been conducted in the fields of the active materials of positive and negative electrodes as well as the additives of nonaqueous electrolyte solutions.

For example, Patent Document 1 discloses a study for improvement of the capacity retention rate in a cycle test by incorporating a sulfonate ester compound and a compound having two or more unsaturated bonds at terminals into a nonaqueous electrolyte solution.

Patent Document 2 discloses a study for improvement of the capacity retention rate in a cycle test and enhancement of the flame retardancy of an electrolyte solution by incorporating a specific phosphite ester compound and a compound having one polymerizable functional group in the molecule into a nonaqueous electrolyte solution.

Patent Document 3 discloses a study for improvement of the capacity retention rate in a cycle test as well as battery swelling, which improvement is attained by using a nonaqueous electrolyte solution that contains an isocyanate compound and an imide salt in combination with a positive electrode that contains a certain amount of water and thereby allowing the isocyanate compound to reduce the water content in the positive electrode while allowing the isocyanate compound and the imide salt to form a coating film suitable for a negative electrode.

Patent Document 4 discloses a study for improvement of battery swelling in an 85°C storage test by incorporating a specific isocyanate compound into a nonaqueous electrolyte solution.

Patent Document 5 discloses nonaqueous electrolyte solutions comprising triallyl isocyanurate (0.5 wt%), vinylene carbonate (0.5 wt%) and $LiPO_2F_2$ (0.5 wt%). Methylene methane disulfonate is disclosed as optional electrolyte additive.

Patent Document 6 discloses nonaqueous electrolyte solutions comprising 0.5 or 1.0 wt% triallyl isocyanurate together with 2 wt% vinylene carbonate. Cyclic sulfonic acid esters such as methylene methane disulfonate is disclosed as optional electrolyte additive.

Patent Document 7 discloses nonaqueous electrolyte solutions comprising methylene methane disulfonate (1 wt%) and vinylene carbonate (1 wt%).

Patent Document 8 discloses nonaqueous electrolyte solutions comprising electrolyte additives triallyl isocyanurate, vinylene carbonate and 1,3-bis(isocyanatomethyl)cyclohexane.

CITATION LIST

PATENT DOCUMENTS

[Patent Document 1] WO 2013/146819
[Patent Document 2] Japanese Unexamined Patent Application Publication No. 2010-282906
[Patent Document 3] Japanese Unexamined Patent Application Publication No. 2011-44339
[Patent Document 4] Japanese Unexamined Patent Application Publication No. 2011-48987
[Patent Document 5] EP3051618 A1
[Patent Document 6] JP 2016 054143 A
[Patent Document 7] US 2004/043300 A1
[Patent Document 8] US 2015/364794 A1

## SUMMARY OF INVENTION

### Technical Problem

In recent years, improvement in the capacity of lithium secondary batteries that are used as power sources to be mounted on electric vehicles has been accelerated, and voids other than constituents inside battery casings have been reduced. Thus, a gas generated during high-temperature storage causes swelling of such batteries. In addition, since the amount of an electrolyte solution in these batteries are small, for example, the amount of a repair component used in the event of elution of a coating film deposited on an electrode due to high-temperature storage is limited. Accordingly, elution of the coating film presents two problems of an increase in the thickness of the coating film and an increase in the battery resistance that are caused by progress of continuous decomposition reaction of the electrolyte solution. Therefore, it is important to inhibit not only the generation of a gas during high-temperature storage but also an increase in the battery resistance.

However, according to the studies conducted by the present inventor, the use of an electrolyte solution that contains a compound having one polymerizable functional group in the molecule and/or a specific isocyanate compound as disclosed in Patent Documents 1 to 4 has a problem of increasing the battery resistance, although it inhibits the generation of a gas during high-temperature storage.

### Solution to Problem

The present inventor intensively studied to solve the above-described problems and consequently discovered that not only the generation of a gas during high-temperature storage but also an increase in the battery resistance can be inhibited by using a nonaqueous electrolyte solution which contains a compound represented by the below-described Formula (A), a cyclic carbonate having an unsaturated carbon-carbon bond, and at least one compound selected from the group consisting of compounds represented by the below-described Formula (B) or (C), and in which the amounts of these compounds are suitably adjusted, thereby arriving at the present invention.

That is, the present invention provides, for example, the following specific modes of [1] to [8].

[1] A nonaqueous electrolyte solution, containing: a compound represented by the following Formula (A); a cyclic carbonate having an unsaturated carbon-carbon bond; and at least one compound selected from the group consisting of a compound represented by the following Formula (B) and a compound represented by the following Formula (C), wherein

the content of the cyclic carbonate having the unsaturated carbon-carbon bond with respect to a total amount of the nonaqueous electrolyte solution is 0.01% by mass or higher and 1.5% by mass or less,

when the nonaqueous electrolyte solution contains only one of the compounds represented by Formula (B) and the compound represented by Formula (C), the content of the compound represented by Formula (B) or (C) with respect to the total amount of the nonaqueous electrolyte solution is 0.01% by mass or higher and 0.49% by mass or less, and

when the nonaqueous electrolyte solution contains both of the compound represented by Formula (B) and the compound represented by Formula (C), a total content of the compound represented by Formula (B) and the compound represented by Formula (C) with respect to the total amount of the nonaqueous electrolyte solution is 0.01% by mass or higher and 0.80% by mass or less:

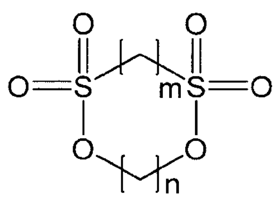

(A)

(wherein, m and n each independently represent an integer of 1 to 3)

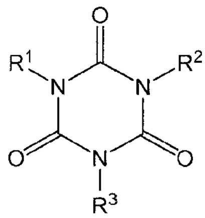

(B)

(wherein, $R^1$ to $R^3$ are optionally the same or different from each other and each represent a hydrocarbon group having 1 to 10 carbon atoms which optionally has a substituent, with the proviso that at least one of $R^1$ to $R^3$ is a hydrocarbon group having an unsaturated carbon-carbon bond)

OCN-Q-NCO (C)

(wherein, Q represents a hydrocarbon group having 3 to 20 carbon atoms, and the hydrocarbon group contains a cycloalkylene group).

[2] The nonaqueous electrolyte solution according to [1], wherein, in Formula (B), the hydrocarbon group having an unsaturated carbon-carbon bond is an allyl group or a methallyl group.
[3] The nonaqueous electrolyte solution according to [1] or [2], further containing a fluorine atom-containing cyclic carbonate.
[4] The nonaqueous electrolyte solution according to [3], wherein the content of the fluorine atom-containing cyclic carbonate is 0.01% by mass or higher and 5% by mass or less with respect to a total amount of the nonaqueous electrolyte solution.
[5] The nonaqueous electrolyte solution according to any one of [1] to [4], further containing at least one salt selected from the group consisting of a fluorinated salt and an oxalate salt.
[6] The nonaqueous electrolyte solution according to [5], wherein the content of the fluorinated salt and/or the oxalate salt is 0.01% by mass or higher and 5% by mass or less with respect to a total amount of the nonaqueous electrolyte solution.
[7] A nonaqueous electrolyte battery, containing: a positive electrode and a negative electrode, which are capable of absorbing and releasing metal ions; and a nonaqueous electrolyte solution,
wherein the nonaqueous electrolyte solution is the nonaqueous electrolyte solution according to any one of [1] to [6].
[8] The nonaqueous electrolyte battery according to [7], wherein a positive electrode active material contained in the positive electrode is a metal oxide represented by the following composition formula (1):

$$Li_{a1}Ni_{b1}CO_{c1}Md_{1}O_{2} \quad (1)$$

(wherein, a1, b1, c1 and d1 represent numerical values of $0.90 \leq a1 \leq 1.10$, $0.50 \leq b1 \leq 0.98$, $0.01 \leq c1 < 0.50$ and $0.01 \leq d1 < 0.50$, satisfying $b1 + c1 + d1 = 1$; and M represents at least one element selected from the group consisting of Mn, Al, Mg, Zr, Fe, Ti, and Er).
[9] The nonaqueous electrolyte battery according to [7] or [8], wherein the negative electrode contains a negative electrode active material that contains metal particles alloyable with Li, and graphite.
[10] The nonaqueous electrolyte battery according to [9], wherein the metal particles alloyable with Li are metal particles containing at least one metal selected from the group consisting of Si, Sn, As, Sb, Al, Zn, and W.
[11] The nonaqueous electrolyte battery according to [9], wherein the metal particles alloyable with Li are composed of Si or Si metal oxide.
[12] The nonaqueous electrolyte battery according to any one of [9] to [11], wherein the negative electrode active material that contains the metal particles alloyable with Li and the graphite is a composite and/or a mixture of metal particles and graphite particles.
[13] The nonaqueous electrolyte battery according to any one of [9] to [12], wherein the content of the metal particles alloyable with Li is 0.1% by mass or higher and 25% by mass or less with respect to a total amount of the negative electrode active material that contains the metal particles alloyable with Li and the graphite.

Advantageous Effects of Invention

The use of the nonaqueous electrolyte solution of the present invention enables to obtain a nonaqueous electrolyte battery in which not only the generation of a gas during high-temperature storage but also an increase in the resistance of the nonaqueous electrolyte battery (hereinafter, simply referred to as "battery resistance") can be inhibited.

DESCRIPTION OF EMBODIMENTS

Modes for carrying out the present invention will now be described in detail. The below-described modes are merely examples (representative examples) of the embodiments the present invention, and the present invention is not restricted thereto. Further, modifications can be arbitrarily made to carry out the present invention, without departing from the gist of the present invention.

<1. Nonaqueous Electrolyte Solution>

<1-1. Compound Represented by Formula (A)>

The nonaqueous electrolyte solution of the present invention contains a compound represented by the following Formula (A):

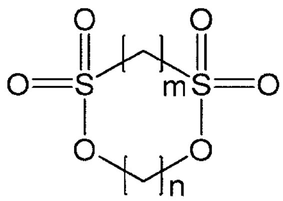

(A)

(wherein, m and n each independently represent an integer of 1 to 3).

In Formula (A), specific examples of a combination of m and n (m, n) include (1,1), (1,2), (1,3), (2,1), (2,2), (2,3), (3,1), (3,2), and (3,3).

Thereamong, the combination of m and n (m,n) is, for example, preferably (1,1), (1,2), (1,3), (2,1), (2,2) or (3,1), more preferably (1,1), (1,2), (2,1) or (2,2), still more preferably (1,1) or (1,2).

The compound represented by Formula (A) may be used singly, or two or more thereof may be used in any combination at any ratio. The content of the compound represented by Formula (A) with respect to a total amount of the nonaqueous electrolyte solution of the present invention is not particularly restricted and may be set arbitrarily as long as the effects of the present invention are not markedly impaired; however, in 100% by mass of the nonaqueous electrolyte solution, the content of the compound represented by Formula (A) is usually 0.001% by mass or higher, preferably 0.01% by mass or higher, more preferably 0.1% by mass or higher, but usually 5% by mass or less, preferably 4% by mass or less, more preferably 3% by mass or less, particularly preferably 2% by mass or less. When the content of the compound represented by Formula (A) is in this range, an increase in the battery resistance during high-temperature storage can be favorably inhibited. When two or more compounds represented by Formula (A) are used in combination, a total amount thereof should satisfy the above-described range.

<1-2. Cyclic Carbonate Compound Having Unsaturated Carbon-Carbon Bond>

The cyclic carbonate having an unsaturated carbon-carbon bond (hereinafter, may be referred to as "unsaturated cyclic carbonate") contained in the nonaqueous electrolyte solution of the present invention is not particularly restricted as long as it is a cyclic carbonate having a carbon-carbon double bond or a carbon-carbon triple bond, and any such unsaturated carbonate can be used. It is noted here that the term "unsaturated cyclic carbonate" used herein also encompasses a cyclic carbonate having an aromatic ring.

Examples of the unsaturated cyclic carbonate include: vinylene carbonates; ethylene carbonates substituted with a substituent having an aromatic ring, a carbon-carbon double bond or a carbon-carbon triple bond; phenyl group-containing cyclic carbonates; vinyl group-containing cyclic carbonates; allyl group-containing cyclic carbonates; and catechol group-containing cyclic carbonates. The unsaturated cyclic carbonate is preferably a vinylene carbonate, a vinyl group-containing ethylene carbonate, an allyl group-containing ethylene carbonate, or a phenyl group-containing ethylene carbonate.

Examples of the vinylene carbonates include vinylene carbonate, methylvinylene carbonate, 4,5-dimethylvinylene carbonate, phenylvinylene carbonate, 4,5-diphenylvinylene carbonate, vinylvinylene carbonate, 4,5-divinylvinylene carbonate, allylvinylene carbonate, 4,5-diallylvinylene carbonate, 4-fluorovinylene carbonate, 4-fluoro-5-methylvinylene carbonate, 4-fluoro-5-phenylvinylene carbonate, 4-fluoro-5-vinylvinylene carbonate, and 4-allyl-5-fluorovinylene

carbonate.

Specific examples of the ethylene carbonates substituted with a substituent having an aromatic ring, a carbon-carbon double bond or a carbon-carbon triple bond include vinylethylene carbonate, 4,5-divinylethylene carbonate, 4-methyl-5-vinylethylene carbonate, 4-allyl-5-vinylethylene carbonate, ethynylethylene carbonate, 4,5-diethynylethylene carbonate, 4-methyl-5-ethynylethylene carbonate, 4-vinyl-5-ethynylethylene carbonate, 4-allyl-5-ethynylethylene carbonate, phenylethylene carbonate, 4,5-diphenylethylene carbonate, 4-phenyl-5-vinylethylene carbonate, 4-allyl-5-phenylethylene carbonate, allylethylene carbonate, 4,5-diallylethylene carbonate, and 4-methyl-5-allylethylene carbonate.

Thereamong, the unsaturated cyclic carbonate is preferably, for example, vinylene carbonate, methylvinylene carbonate, 4,5-dimethylvinylene carbonate, vinylvinylene carbonate, 4,5-vinylvinylene carbonate, allylvinylene carbonate, 4,5-diallylvinylene carbonate, vinylethylene carbonate, 4,5-divinylethylene carbonate, 4-methyl-5-vinylethylene carbonate, allylethylene carbonate, 4,5-diallylethylene carbonate, 4-methyl-5-allylethylene carbonate, 4-allyl-5-vinylethylene carbonate, ethynylethylene carbonate, 4,5-diethynylethylene carbonate, 4-methyl-5-ethynylethylene carbonate, or 4-vinyl-5-ethynylethylene carbonate.

Further, vinylene carbonate, vinylethylene carbonate, and ethynylethylene carbonate are particularly preferred since they each yield a stable interface protective film.

The molecular weight of the unsaturated cyclic carbonate is not particularly restricted and may be set arbitrarily as long as the effects of the present invention are not markedly impaired. The molecular weight of the unsaturated cyclic carbonate is preferably 80 to 250. When the molecular weight of the unsaturated cyclic carbonate is in this range, a solubility of the unsaturated cyclic carbonate in the nonaqueous electrolyte solution is likely to be ensured, so that the effects of the present invention are likely to be expressed sufficiently. The molecular weight of the unsaturated cyclic carbonate is more preferably 85 to 150. A method of producing the unsaturated cyclic carbonate is not particularly restricted, and any known method can be selected to produce the unsaturated cyclic carbonate. Alternatively, a commercially available product may be obtained and used.

The unsaturated cyclic carbonate may be used singly, or two or more thereof may be used in any combination at any ratio. The content of the unsaturated cyclic carbonate with respect to the total amount of the nonaqueous electrolyte solution of the present invention is 0.01% by mass or higher, more preferably 0.1% by mass or higher, but 1.5% by mass or less, preferably 1.4% by mass or less, more preferably 1.3% by mass or less, particularly preferably 1.25% by mass or less, in 100% by mass of the nonaqueous electrolyte solution. When the content of the unsaturated cyclic carbonate is in this range, an increase in the battery resistance during high-temperature storage is likely to be avoided. When two or more unsaturated cyclic carbonates are used in combination, a total amount thereof should satisfy the above-described range.

<1-3. Compound Represented by Formula (B)>

The nonaqueous electrolyte solution of the present invention contains at least one compound selected from the group consisting of a compound represented by the following Formula (B) and a compound represented by the below-described Formula (C):

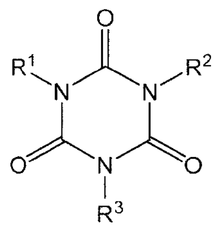

(B)

In Formula (B), $R^1$ to $R^3$ are optionally the same or different from each other and each represent a hydrocarbon group having 1 to 10 carbon atoms which optionally has a substituent, with the proviso that at least one of $R^1$ to $R^3$ is a hydrocarbon group having an unsaturated carbon-carbon bond.

Examples of the substituent include a cyano group, an isocyanate group, an acyl group (-(C=O)-Ra), an acyloxy group (-O(C=O)-Ra), an alkoxycarbonyl group (-(C=O)O-Ra), a sulfonyl group ($-SO_2$-Ra), a sulfonyloxy group ($-O(SO_2)$-Ra), an alkoxysulfonyl group ($-(SO_2)$-O-Ra), an alkoxycarbonyloxy group (-O-(C=O)-O-Ra), an ether group (-O-Ra), an acryl group, a methacryl group, a halogen (preferably fluorine), and a trifluoromethyl group. Ra represents an alkyl group having 1 to 10 carbon atoms, an alkenyl group having 2 to 10 carbon atoms, or an alkynyl group having 2 to 10 carbon atoms.

Among these substituents, a cyano group, an isocyanate group, an acyloxy group (-O(C=O)-Ra), an alkoxycarbonyl group (-(C=O)O-Ra), a sulfonyl group ($-SO_2$-Ra), a sulfonyloxy group ($-O(SO_2)$-Ra), an alkoxysulfonyl group

(-($SO_2$)-O-Ra), an acryl group, or a methacryl group is preferred.

Specific examples of the hydrocarbon group include alkyl groups, cycloalkyl groups, alkenyl groups, alkynyl groups, and aryl groups optionally bound through an alkylene group. Thereamong, the hydrocarbon group is preferably an alkyl group, an alkenyl group or an alkynyl group, more preferably an alkenyl group or an alkynyl group, particularly preferably an alkenyl group.

Specific examples of the alkyl groups include a methyl group, an ethyl group, an *n*-propyl group, an *i*-propyl group, an *n*-butyl group, an s-butyl group, an *i*-butyl group, *t*-butyl group, an *n*-pentyl group, a *t*-amyl group, a hexyl group, a heptyl group, an octyl group, a nonyl group, and a decyl group. Thereamong, an ethyl group, an *n*-propyl group, an *n*-butyl group, an *n*-pentyl group or a hexyl group is preferred, and an ethyl group, an *n*-propyl group or an *n*-butyl group is more preferred.

Specific examples of the cycloalkyl groups include a cyclopentyl group, a cyclohexyl group, a cycloheptyl group, a cyclooctyl group, and an adamantyl group, among which a cyclohexyl group or an adamantyl group is preferred.

Specific examples of the alkenyl groups include a vinyl group, an allyl group, a methallyl group, a 2-butenyl group, a 3-methyl-2-butenyl group, a 3-butenyl group, and a 4-pentenyl group. Thereamong, a vinyl group, an allyl group, a methallyl group or a 2-butenyl group is preferred, a vinyl group, an allyl group or a methallyl group is more preferred, an allyl group or a methallyl group is particularly preferred, and an allyl group is most preferred. When the hydrocarbon group is any of these alkenyl groups, appropriate steric hindrance is attained, and an increase in the electrode resistance due to reaction of the compound of Formula (B) on an electrode can be adjusted at a favorable level.

Specific examples of the alkynyl groups include an ethynyl group, a 2-propynyl group, a 2-butynyl group, a 3-butynyl group, a 4-pentynyl group, and a 5-hexynyl group. Thereamong, an ethynyl group, a 2-propynyl group, a 2-butynyl group or a 3-butynyl group is preferred, a 2-propynyl group or a 3-butynyl group is more preferred, a 2-propynyl group is particularly preferred. When the hydrocarbon group is any of these alkynyl groups, appropriate steric hindrance is attained, and an increase in the electrode resistance due to reaction of the compound of Formula (B) on an electrode can be adjusted at a favorable level.

Specific examples of the aryl groups optionally bound through an alkylene group include a phenyl group, a tolyl group, a benzyl group, and a phenethyl group.

$R^1$ to $R^3$ are each preferably an alkyl group, an allyl group or a methallyl group, which optionally has a substituent and, from the standpoint of the coating film-forming ability, $R^1$ to $R^3$ are each most preferably an allyl group. Further, the hydrocarbon group having an unsaturated carbon-carbon bond may have the unsaturated carbon-carbon bond in a substituent, preferably contains a group having the unsaturated carbon-carbon bond at a terminal, more preferably contains at least one selected from the group consisting of an allyl group, a methallyl group and a butenyl group, still more preferably is an allyl group or a methallyl group.

Specific examples of the compound represented by Formula (B) that is used in the present invention include compounds having the following structures.

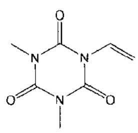 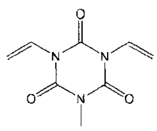 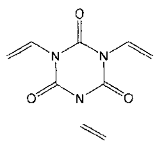 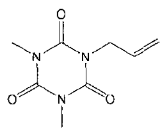 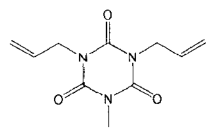

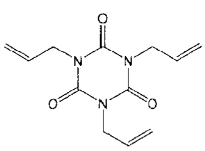 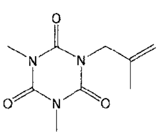 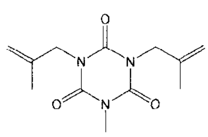 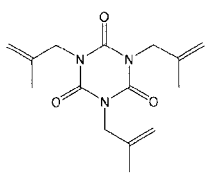

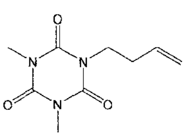 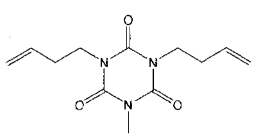 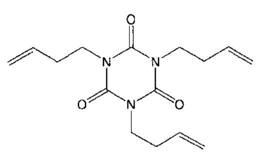

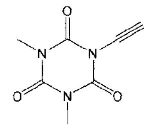 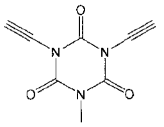 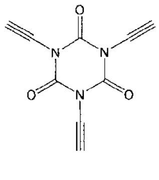 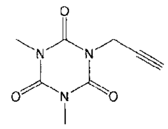 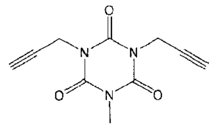

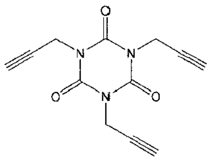 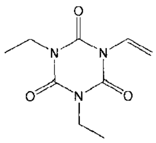 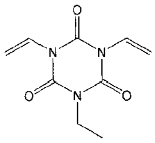 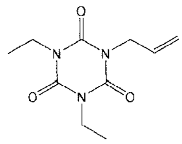 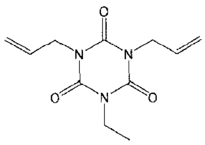

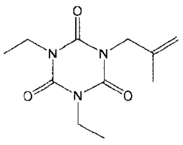 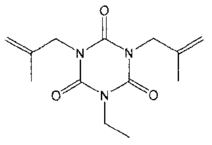 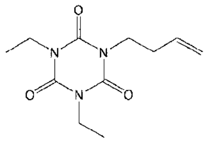 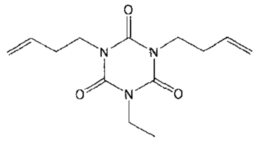

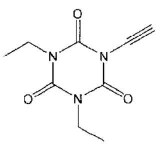 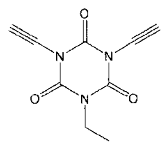 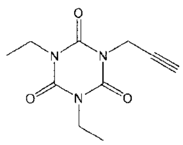 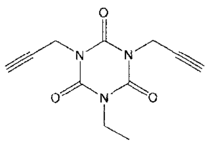

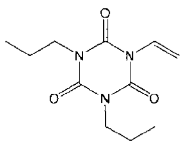 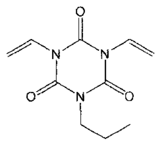 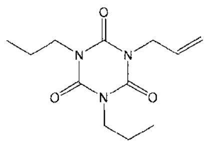 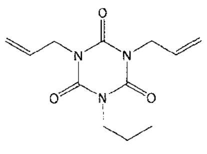

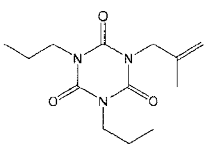 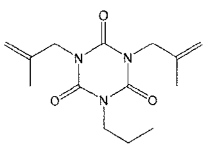 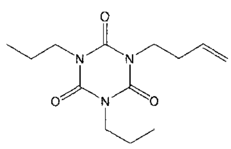 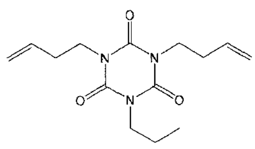

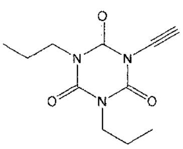 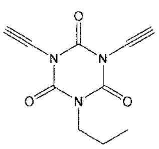 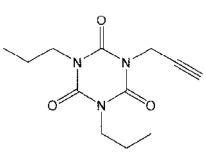 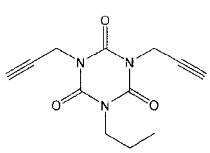

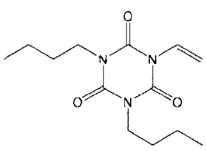 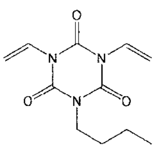 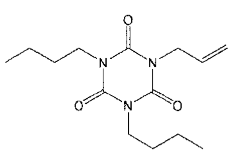 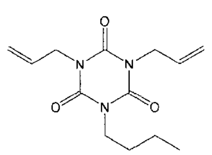

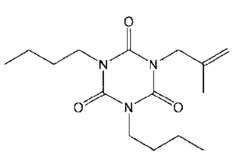 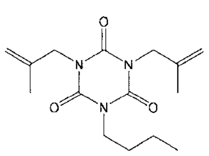 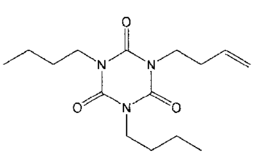 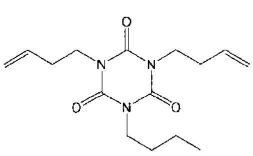

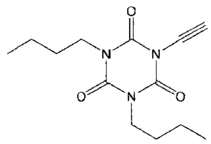
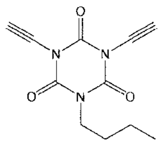
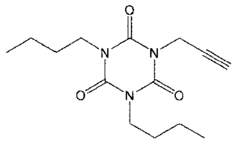
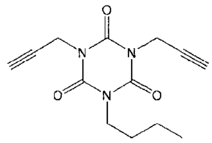

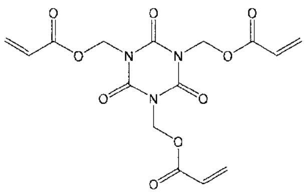
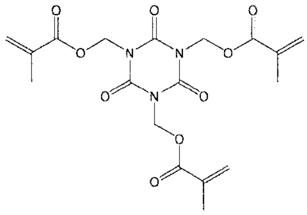
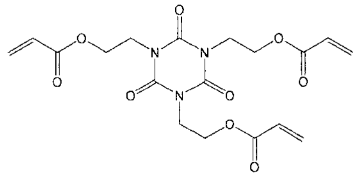

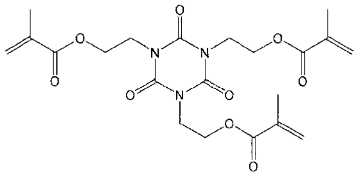
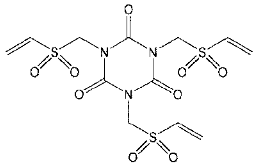
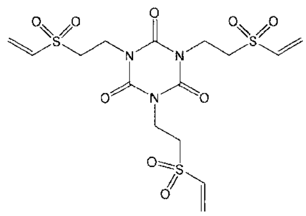

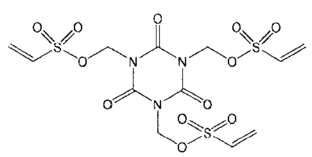
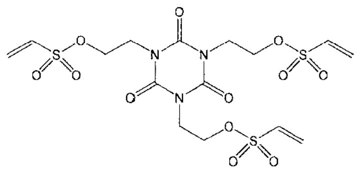

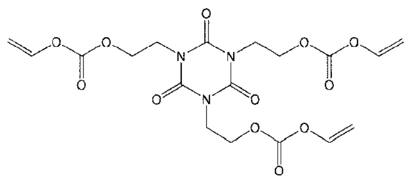
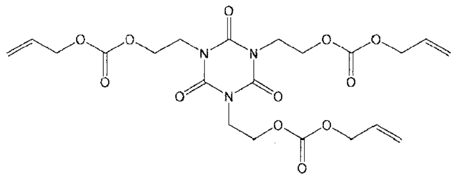

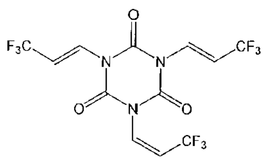
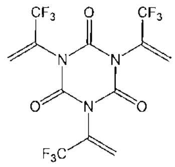
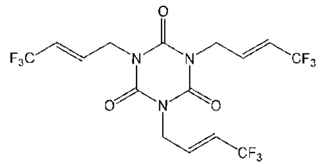
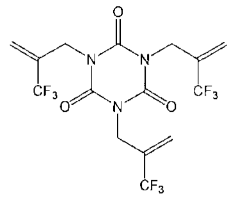

Thereamong, preferred examples include compounds having the following structures.

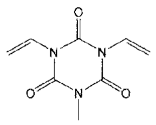
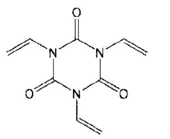
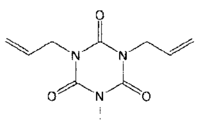
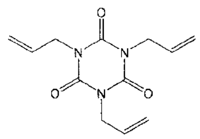

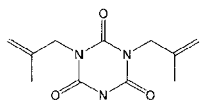
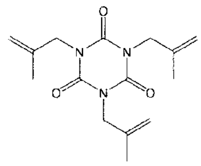
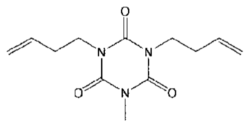
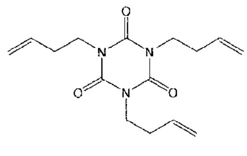

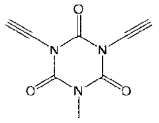
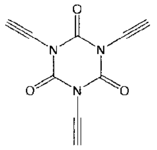
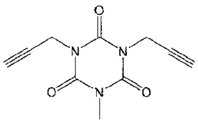
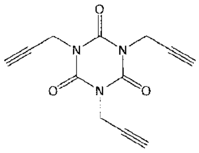
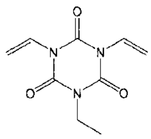

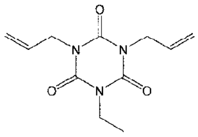
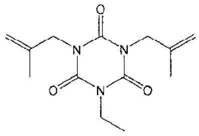
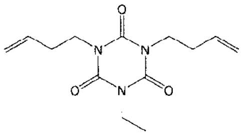
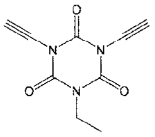

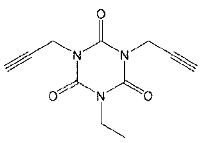
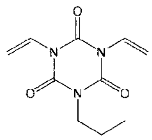
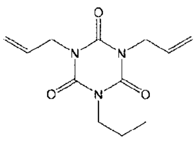
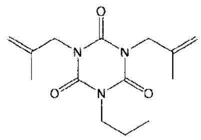

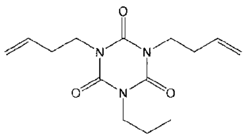
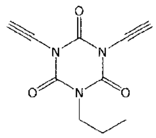
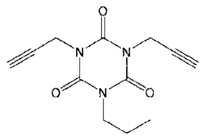
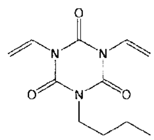

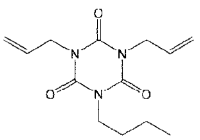
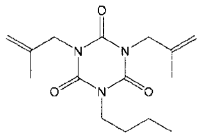
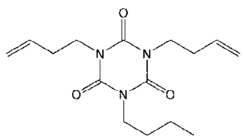
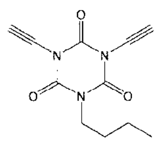

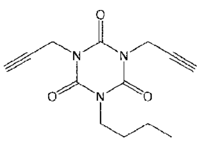
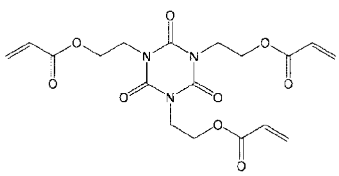
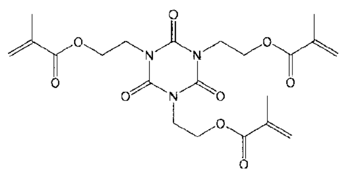

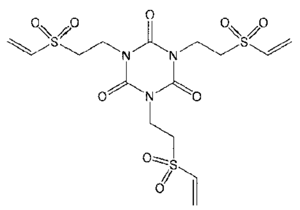
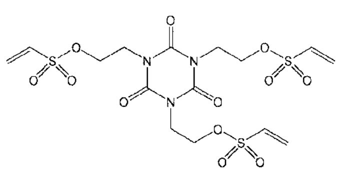

More preferred examples include compounds having the following structures.

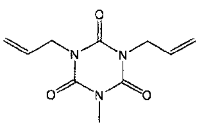
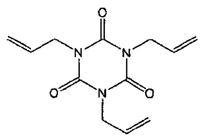
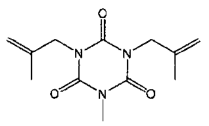
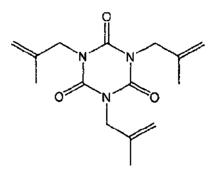

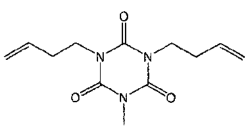
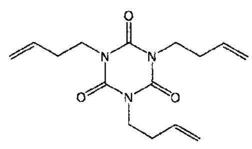
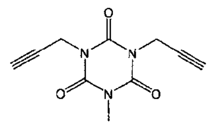
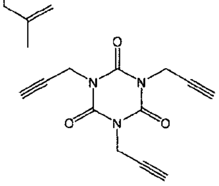

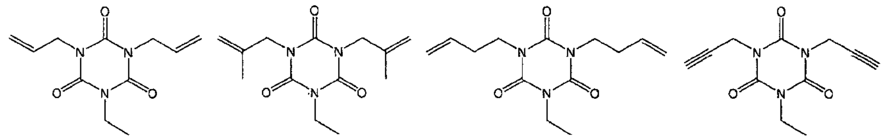

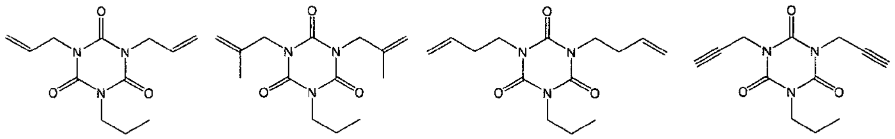

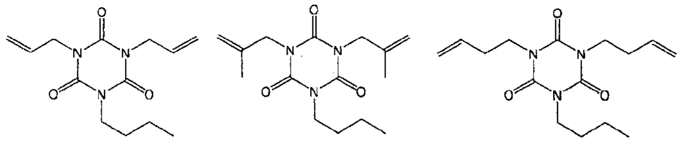

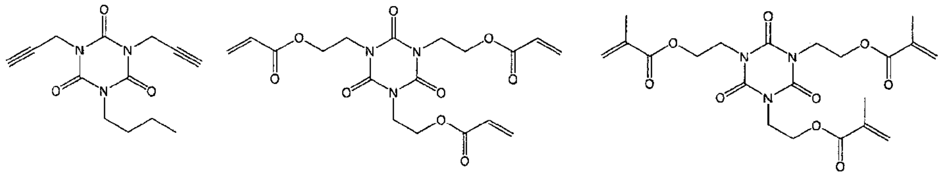

Particularly preferred examples include compounds having the following structures.

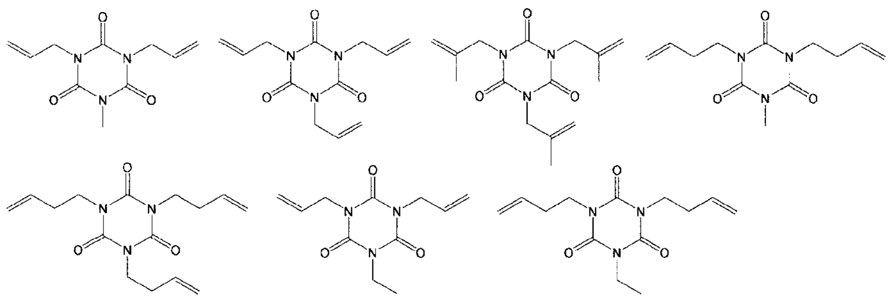

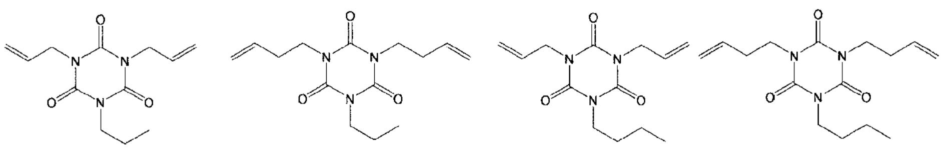

Most preferred examples include compounds having the following structures.

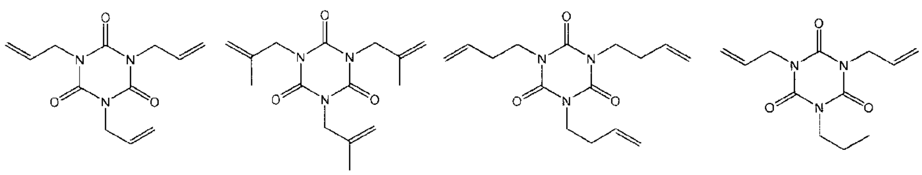

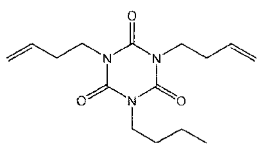

When the nonaqueous electrolyte solution does not contain any compound represented by the below-described Formula (C), the content of the compound represented by Formula (B) with respect to the total amount of the nonaqueous electrolyte solution of the present invention is 0.01% by mass or higher, preferably 0.05% by mass or higher, but 0.49% by mass or less, preferably 0.40% by mass or less, more preferably 0.30% by mass or less, still more preferably 0.25% by mass or less, particularly preferably 0.20% by mass or less, in 100% by mass of the nonaqueous electrolyte solution.

<1-4. Compound Represented by Formula (C)>

The nonaqueous electrolyte solution of the present invention contains at least one compound selected from the group consisting of a compound represented by the above-described Formula (B) and a compound represented by the following Formula (C):

OCN-Q-NCO (C)

In Formula (C), Q represents a hydrocarbon group having 3 to 20 carbon atoms, and the hydrocarbon group contains a cycloalkylene group.

Specific examples of the cycloalkylene group include a cyclopropylene group, a cyclobutylene group, a cyclopentyne group, and a cyclohexylene group. Thereamong, a cyclohexylene group is preferred.

Some of the hydrogen atoms of the hydrocarbon group having 3 to 20 carbon atoms may be substituted with a halogen atom. Among halogen atoms, a fluorine atom is preferred from the standpoint of improving the reactivity on the negative electrode surface.

Specific examples of the compound represented by Formula (C) that is used in the present invention include compounds having the following structures.

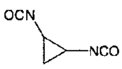 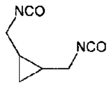 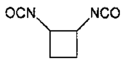 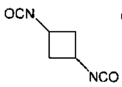 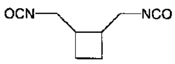 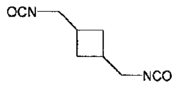

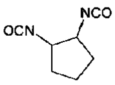 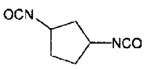 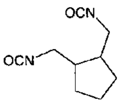 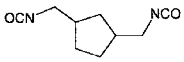 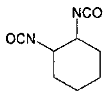 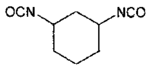

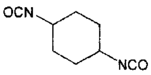 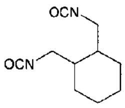 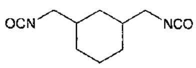 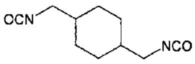 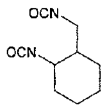

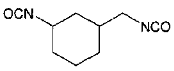 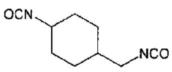 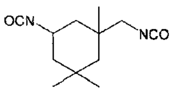 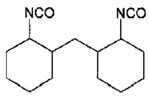

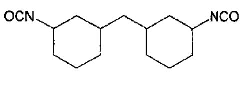 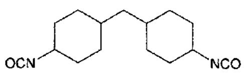 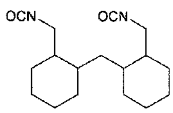

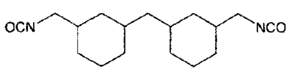 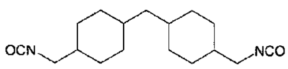

Thereamong, preferred examples include compounds having the following structures.

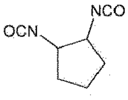
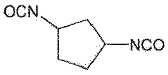
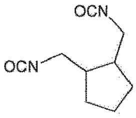
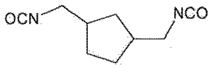
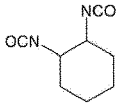
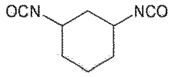
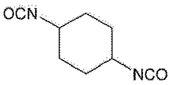
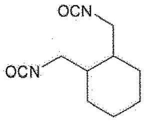
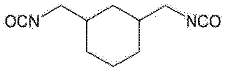
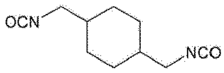
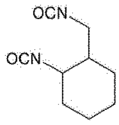
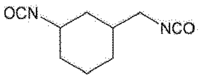
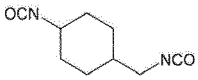
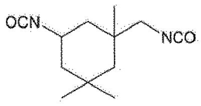
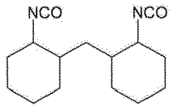
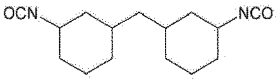
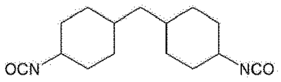
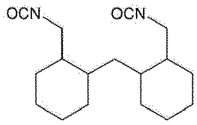
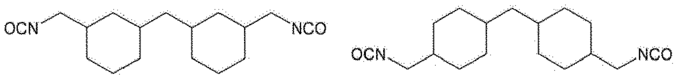

More preferred examples include compounds having the following structures.

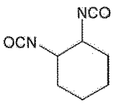
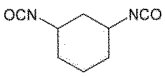
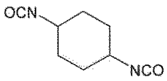
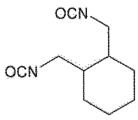
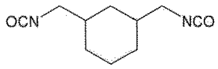
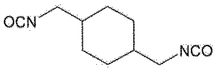
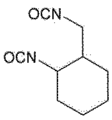
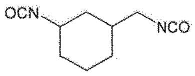
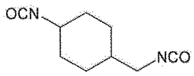
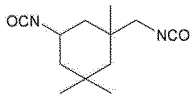
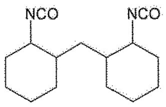
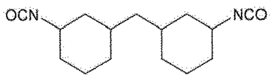
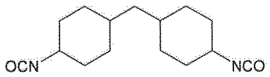
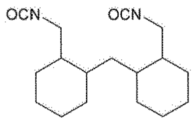
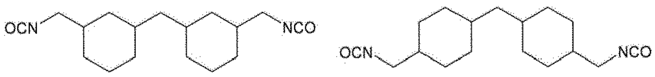

Particularly preferred examples include compounds having the following structures.

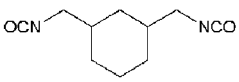
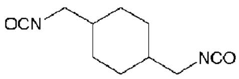
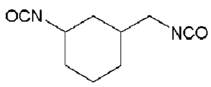
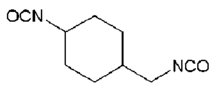
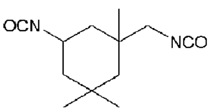
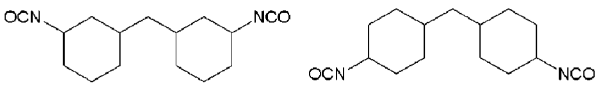

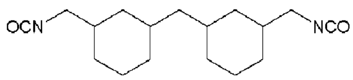 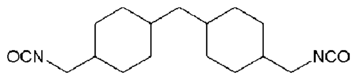

When the nonaqueous electrolyte solution does not contain any compound represented by (B), the content of the compound represented by Formula (C) with respect to the total amount of the nonaqueous electrolyte solution of the present invention is 0.01% by mass or higher, preferably 0.05% by mass or higher, but 0.49% by mass or less, preferably 0.40% by mass or less, more preferably 0.30% by mass or less, still more preferably 0.25% by mass or less, particularly preferably 0.20% by mass or less, in 100% by mass of the nonaqueous electrolyte solution.

When the nonaqueous electrolyte solution of the present invention contains both a compound represented by Formula (B) and a compound represented by Formula (C), a total content of the compound represented by Formula (B) and the compound represented by Formula (C) with respect to the total amount of the nonaqueous electrolyte solution of the present invention is 0.01% by mass or higher, preferably 0.05% by mass or higher, more preferably 0.10% by mass or higher, particularly preferably 0.20% by mass or higher, but 0.80% by mass or less, preferably 0.70% by mass or less, more preferably 0.60% by mass or less, still more preferably 0.50% by mass or less, particularly preferably 0.40% by mass or less, in 100% by mass of the nonaqueous electrolyte solution.

The mechanism in which not only the generation of a gas during high-temperature storage but also an increase in the battery resistance can be inhibited by using a nonaqueous electrolyte solution, which contains a compound represented by Formula (A), a cyclic carbonate having an unsaturated carbon-carbon bond, and at least one compound selected from the group consisting of compounds represented by Formula (B) or (C) and in which the amounts of these compounds are suitably adjusted, is not clear; however, it is presumed as follows.

The compound represented by Formula (A) and the cyclic carbonate having an unsaturated carbon-carbon bond electrochemically react with a negative electrode during initial charging, as a result of which their decomposition reactions proceed. The compound represented by Formula (B) and/or the compound represented by Formula (C) have, in their structures, two or more moieties that react with the resulting reductive decomposition products and, therefore, form a composite coating film, which is composed of the compound represented by Formula (A), the cyclic carbonate having an unsaturated carbon-carbon bond, and the compound represented by Formula (B) and/or the compound represented by Formula (C), on the negative electrode. Since this coating film exhibits high insulation under high temperatures, side reactions on the negative electrode are suppressed during high-temperature storage, so that gas generation is inhibited. However, when the reaction between the reductive decomposition product of the cyclic carbonate having an unsaturated carbon-carbon bond and the compound represented by Formula (B) and/or the compound represented by Formula (C) does not sufficiently proceed during the initial charging, this reaction proceeds excessively during high-temperature storage, causing a problem of an increase in the battery resistance.

To address this problem, in the present invention, the added amount of the cyclic carbonate having an unsaturated carbon-carbon bond and that of the compound represented by Formula (B) and/or the compound represented by Formula (C) are suitably adjusted so as to allow the above-described reaction to sufficiently proceed during the initial charging, whereby an excessive reaction between the reductive decomposition product and the compound represented by Formula (B) and/or the compound represented by Formula (C) during high-temperature storage is successfully controlled. Further, the reaction site of the compound represented by Formula (B) and that of the compound represented by Formula (C) are different from each other in terms of reactive species. For example, it is assumed that the compound represented by Formula (B) reacts with a radical species generated by reductive decomposition of the cyclic carbonate having an unsaturated carbon-carbon bond, while the compound represented by Formula (C) reacts with an anionic species. Accordingly, when the compound represented by Formula (B) and the compound represented by Formula (C) are used in combination, both of the radical species and the anionic species of the reductive decomposition product are involved in a coating film-forming reaction; therefore, a more preferred coating film is formed as compared to a case where the compound represented by Formula (B) or (C) is added alone. It was discovered that, consequently, not only the inhibition of gas generation during high temperature storage but also the inhibition of an increase in the battery resistance are improved.

<1-5. Fluorine Atom-Containing Cyclic Carbonate>

The nonaqueous electrolyte solution of the present invention preferably further contain a fluorine atom-containing cyclic carbonate.

Examples of the fluorine atom-containing cyclic carbonate include fluorinated products of cyclic carbonates having an alkylene group having 2 to 6 carbon atoms and derivatives thereof, such as fluorinated ethylene carbonates and derivatives thereof. Examples of the derivatives of fluorinated ethylene carbonates include fluorinated products of ethylene carbonates substituted with an alkyl group (e.g., an alkyl group having 1 to 4 carbon atoms). Thereamong, ethylene carbonates having 1 to 8 fluorine atoms and derivatives thereof are preferred.

Specific examples thereof include monofluoroethylene carbonate, 4,4-difluoroethylene carbonate, 4,5-difluoroethylene carbonate, 4-fluoro-4-methylethylene carbonate, 4,5-difluoro-4-methylethylene carbonate, 4-fluoro-5-methylethylene carbonate, 4,4-difluoro-5-methylethylene carbonate, 4-(fluoromethyl)-ethylene carbonate, 4-(difluoromethyl)-ethylene carbonate, 4-(trifluoromethyl)-ethylene carbonate, 4-(fluoromethyl)-4-fluoroethylene carbonate, 4-(fluoromethyl)-5-fluoroethylene carbonate, 4-fluoro-4,5-dimethylethylene carbonate, 4,5-difluoro-4,5-dimethylethylene carbonate, and 4,4-difluoro-5,5-dimethylethylene carbonate.

Thereamong, at least one selected from the group consisting of monofluoroethylene carbonate, 4,4-difluoroethylene carbonate and 4,5-difluoroethylene carbonate is preferred from the standpoints of imparting the nonaqueous electrolyte solution with a high ionic conductivity and favorably forming an interface protective film.

Any of the above-described fluorine atom-containing cyclic carbonate compounds may be used singly, or two or more thereof may be used in any combination at any ratio. The content of the fluorine atom-containing cyclic carbonate with respect to the total amount of the nonaqueous electrolyte solution of the present invention is not restricted and may be set arbitrarily as long as the effects of the present invention are not markedly impaired; however, it is usually 0.001% by mass or higher, preferably 0.01% by mass or higher, more preferably 0.1% by mass or higher, still more preferably 0.5% by mass or higher, particularly preferably 1% by mass or higher, but usually 50.0% by mass or less, preferably 30.0% by mass or less, more preferably 20.0% by mass or less, still more preferably 10.0% by mass or less, in 100% by mass of the nonaqueous electrolyte solution. When two or more fluorine atom-containing cyclic carbonate compounds are used in combination, a total amount thereof should satisfy the above-described range.

<1-6. Fluorinated Salt and Oxalate Salt>

The nonaqueous electrolyte solution of the present invention preferably further contain at least one salt selected from the group consisting of a fluorinated salt and an oxalate salt.

<1-6-1. Fluorinated Salt>

There is no particularly restriction on a fluorinated salt that can be used in the nonaqueous electrolyte solution of the present invention; however, a difluorophosphate salt, a fluorosulfonate salt, and a salt having a bis-fluorosulfonylimide structure are preferred since these salts contain a highly dissociable fluorine atom in their structures and are thus capable of, for example, forming a composite coating film by preferably reacting with an anion (nucleophile) generated by the compound represented by Formula (A) through a reduction reaction. A difluorophosphate salt and a difluorosulfonate salt are more preferred since their fluorine atoms are particularly highly dissociable and their reactions with the nucleophile proceed in a preferred manner. These salts will now be described.

<<Difluorophosphate>>

A counter cation of the difluorophosphate salt is not particularly restricted, and examples thereof include lithium, sodium, potassium, rubidium, cesium, magnesium, calcium, barium, and ammonium represented by $NR^{13}R^{14}R^{15}R^{16}$ (wherein, $R^{13}$ to $R^{16}$ each independently represent a hydrogen atom or an organic group having 1 to 12 carbon atoms).

The organic group having 1 to 12 carbon atoms that is represented by $R^{13}$ to $R^{16}$ of the above-described ammonium is not particularly restricted, and examples thereof include alkyl groups optionally substituted with a halogen atom, cycloalkyl groups optionally substituted with a halogen atom or an alkyl group, aryl groups optionally substituted with a halogen atom or an alkyl group, and nitrogen atom-containing heterocyclic groups optionally having a substituent. Thereamong, $R^{13}$ to $R^{16}$ are preferably each independently a hydrogen atom, an alkyl group, a cycloalkyl group, or a nitrogen atom-containing heterocyclic group.

Specific examples of the difluorophosphate salt include lithium difluorophosphate, sodium difluorophosphate, and potassium difluorophosphate, among which lithium difluorophosphate is preferred.

Any of these difluorophosphate salts may be used singly, or two or more thereof may be used in any combination at any ratio. Further, the amount of the difluorophosphate salt to be incorporated is not particularly restricted and may be set arbitrarily as long as the effects of the present invention are not markedly impaired.

The content of the difluorophosphate salt with respect to the total amount of the nonaqueous electrolyte solution of the present invention is usually 0.001% by mass or higher, preferably 0.01% by mass or higher, more preferably 0.1% by mass or higher, but usually 10% by mass or less, preferably 5% by mass or less, more preferably 3% by mass or less, still more preferably 2% by mass or less, most preferably 1% by mass or less, in 100% by mass of the nonaqueous electrolyte solution. When two or more difluorophosphate salts are used in combination, a total amount thereof should satisfy the above-described range.

When the content of the difluorophosphate salt(s) is in this range, swelling of a nonaqueous electrolyte battery caused by charging and discharging can be inhibited in a preferred manner.

<<Fluorosulfonate>>

A counter cation of the fluorosulfonate salt is the same as that of the above-described difluorophosphate salt.

Specific examples of the fluorosulfate salt include lithium fluorosulfonate, sodium fluorosulfonate, potassium fluorosulfonate, rubidium fluorosulfonate, and cesium fluorosulfonate, among which lithium fluorosulfonate is preferred.

Any of these fluorosulfonate salts may be used singly, or two or more thereof may be used in any combination at any ratio. Further, the content of the fluorosulfonate salt with respect to the total amount of the nonaqueous electrolyte solution of the present invention is usually 0.001% by mass or higher, preferably 0.01% by mass or higher, more preferably 0.1% by mass or higher, but usually 10% by mass or less, preferably 5% by mass or less, more preferably 3% by mass or less, still more preferably 2% by mass or less, most preferably 1% by mass or less, in 100% by mass of the nonaqueous electrolyte solution. When two or more fluorosulfonate salts are used in combination, a total amount thereof should satisfy the above-described range.

When the content of the fluorosulfonate salt(s) is in this range, swelling of a nonaqueous electrolyte battery caused by charging and discharging can be inhibited in a preferred manner.

«Salt Having Bis-Fluorosulfonylimide Structure»

A counter cation of the salt having a bis-fluorosulfonylimide structure is the same as that of the above-described difluorophosphate salt.

Examples of the salt having a bis-fluorosulfonylimide structure include lithium bis-fluorosulfonylimide, sodium bis-fluorosulfonylimide, and potassium bis-fluorosulfonylimide, among which lithium bis-fluorosulfonylimide is preferred.

The content of the salt having a bis-fluorosulfonylimide structure with respect to the total amount of the nonaqueous electrolyte solution of the present invention is usually 0.001% by mass or higher, preferably 0.01% by mass or higher, more preferably 0.1% by mass or higher, but usually 10% by mass or less, preferably 5% by mass or less, more preferably 3% by mass or less, in 100% by mass of the nonaqueous electrolyte solution. When two or more salts having a bis-fluorosulfonylimide structure are used in combination, a total amount thereof should satisfy the above-described range.

When the content of the salt(s) having a bis-fluorosulfonylimide structure is in this range, swelling of a nonaqueous electrolyte battery caused by charging and discharging can be inhibited in a preferred manner.

<1-6-2. Oxalate Salt>

A counter cation of the oxalate salt is the same as that of the above-described difluorophosphate salt.

Specific examples of the oxalate salt include lithium difluoro(oxalato)borate, lithium bis(oxalato)borate, lithium tetrafluoro(oxalato)phosphate, lithium difluoro-bis(oxalato)phosphate, and lithium tris(oxalato)phosphate, among which lithium bis(oxalato)borate and lithium difluoro-bis(oxalato)phosphate are preferred.

Any of these oxalate salts may be used singly, or two or more thereof may be used in any combination at any ratio. Further, the content of the oxalate salt with respect to the total amount of the nonaqueous electrolyte solution of the present invention is usually 0.001% by mass or higher, preferably 0.01% by mass or higher, more preferably 0.1% by mass or higher, but usually less than 8% by mass, preferably 5% by mass or less, more preferably 3% by mass or less, still more preferably 2% by mass or less, most preferably 1.5% by mass or less, in 100% by mass of the nonaqueous electrolyte solution. When two or more oxalate salts are used in combination, a total amount thereof should satisfy the above-described range.

When the content of the oxalate salt(s) is in this range, a nonaqueous electrolyte battery is likely to exhibit a sufficient cycle characteristics-improving effect, and a situation where the high-temperature storage characteristics are deteriorated, the amount of gas generation is increased and the discharge capacity retention rate is reduced is likely to be avoided.

<1-7. Electrolyte>

Similarly to a general nonaqueous electrolyte solution, the nonaqueous electrolyte solution of the present invention usually contains an electrolyte as its component. The electrolyte used in the nonaqueous electrolyte solution of the present invention is not particularly restricted, and any known electrolyte can be used. Specific examples of the electrolyte will now be described in detail.

<Lithium Salt>

As the electrolyte in the nonaqueous electrolyte solution of the present invention, a lithium salt is usually used.

The lithium salt is not particularly restricted as long as it is known to be used in this application, and any lithium salt, specific examples of which include the followings, can be used.

Examples of the lithium salt include:

inorganic lithium salts, such as $LiBF_4$, $LiClO_4$, $LiAlF_4$, $LiSbF_6$, $LiTaF_6$, and $LiWF_7$;
lithium fluorophosphates, such as $LiPF_6$;
lithium tungstates, such as $LiWOF_5$;
lithium carboxylates, such as $HCO_2Li$, $CH_3CO_2Li$, $CH_2FCO_2Li$, $CHF_2CO_2Li$, $CF_3CO_2Li$, $CF_3CH_2CO_2Li$, $CF_3CF_2CO_2Li$, $CF_3CF_2CF_2CO_2Li$, and $CF_3CF_2CF_2CF_2CO_2Li$;
lithium sulfonates, such as $CH_3SO_3Li$;
lithium imide salts, such as $LiN(FCO_2)_2$, $LiN(FCO)(FSO_2)$, $LiN(FSO_2)_2$, $LiN(FSO_2)(CF_3SO_2)$, $LiN(CF_3SO_2)_2$, $LiN(C_2F_5SO_2)_2$, lithium cyclic 1,2-perfluoroethane disulfonylimide, lithium cyclic 1,3-perfluoropropane disulfonylimide, and $LiN(CF_3SO_2)(C_4F_9SO_2)$;
lithium methide salts, such as $LiC(FSO_2)_3$, $LiC(CF_3SO_2)_3$, and $LiC(C_2F_5SO_2)_3$;
lithium oxalate salts, such as lithium difluorooxalatoborate, lithium bis(oxalato)borate, lithium tetrafluoro(oxalato)phosphate, lithium difluorobis(oxalato)phosphate, and lithium tris(oxalato)phosphate; and
fluorine-containing organic lithium salts, such as $LiPF_4(CF_3)_2$, $LiPF_4(C_2F_5)_2$, $LiPF_4(CF_3SO_2)_2$, $LiPF_4(C_2F_5SO_2)_2$, LiBF3CF3, LiBF3C2F5, LiBF3C3F7, LiBF2(CF3)2, $LiBF_2(C_2F_5)_2$, $LiBF_2(CF_3SO_2)_2$, and $LiBF_2(C_2F_5SO_2)_2$.

From the standpoint of further enhancing the effects of improving the charge-discharge rate characteristics and the impedance characteristics in addition to the effect of inhibiting gas generation during high-temperature storage that is attained in the present invention, the lithium salt is preferably one selected from inorganic lithium salts, lithium fluorophosphates, lithium sulfonates, lithium imide salts, and lithium oxalate salts.

Among these lithium salts, for example, $LiPF_6$, $LiBF_4$, $LiSbF_6$, $LiTaF_6$, $LiN(FSO_2)_2$, $LiN(FSO_2)(CF_3SO_2)$, $LiN(CF_3SO_2)_2$, $LiN(C_2F_5SO_2)_2$, lithium cyclic 1,2-perfluoroethane disulfonylimide, lithium cyclic 1,3-perfluoropropane disulfonylimide, $LiC(FSO_2)_3$, $LiC(CF_3SO_2)_3$, $LiC(C_2F_5SO_2)_3$, lithium difluorooxalatoborate, lithium bis(oxalato)borate, lithium tetrafluoro(oxalato)phosphate, lithium difluorobis(oxalato)phosphate, and lithium tris(oxalato)phosphate are particularly preferred because of their effects of improving the low-temperature output characteristics, the high-rate charge-discharge characteristics, the impedance characteristics, the high-temperature storage characteristics, the cycle characteristics and the like. The above-described electrolyte salts may be used singly, or in combination of two or more thereof.

A total concentration of these electrolytes in the nonaqueous electrolyte solution is not particularly restricted; however, it is usually 8% by mass or higher, preferably 8.5% by mass or higher, more preferably 9% by mass or higher, with respect to a total amount of the nonaqueous electrolyte solution. The upper limit of the total concentration is usually 18% by mass or lower, preferably 17% by mass or lower, more preferably 16% by mass or lower. When the total concentration of the electrolytes is in this range, the nonaqueous electrolyte solution has an electrical conductivity appropriate for battery operation, so that sufficient output characteristics tend to be attained.

<1-8. Nonaqueous Solvent>

Similarly to a general nonaqueous electrolyte solution, the nonaqueous electrolyte solution of the present invention usually contains, as its main component, a nonaqueous solvent that dissolves the above-described electrolyte. The nonaqueous solvent used in this embodiment is not particularly restricted, and any known organic solvent can be used. The organic solvent may be, for example, but not particularly limited to: a saturated cyclic carbonate, a linear carbonate, an ether-based compound, or a sulfone-based compound. These organic solvents may be used singly, or in combination of two or more thereof.

<1-8-1. Saturated Cyclic Carbonate>

The saturated cyclic carbonate is usually, for example, an alkylene group having 2 to 4 carbon atoms and, from the standpoint of attaining an improvement in the battery characteristics that is attributed to an increase in the degree of lithium ion dissociation, a saturated cyclic carbonate having 2 to 3 carbon atoms is preferably used.

Examples of the saturated cyclic carbonate include ethylene carbonate, propylene carbonate, and butylene carbonate. Thereamong, ethylene carbonate and propylene carbonate are preferred, and ethylene carbonate, which is unlikely to be oxidized or reduced, is more preferred. Any of these saturated cyclic carbonates may be used singly, or two or more thereof may be used in any combination at any ratio.

The content of the saturated cyclic carbonate is not particularly restricted and may be set arbitrarily as long as the effects of the present invention are not markedly impaired; however, when a single saturated cyclic carbonate is used alone, the lower limit of the content is usually 3% by volume or higher, preferably 5% by volume or higher, with

respect to a total solvent amount of the nonaqueous electrolyte solution. By controlling the content of the saturated cyclic carbonate to be in this range, a decrease in the electrical conductivity caused by a reduction in the dielectric constant of the nonaqueous electrolyte solution is avoided, so that the high-current discharge characteristics of the nonaqueous electrolyte battery, the stability to the negative electrode, and the cycle characteristics are all likely to be attained in favorable ranges. Meanwhile, the upper limit of the content of the saturated cyclic carbonate is usually 90% by volume or less, preferably 85% by volume or less, more preferably 80% by volume or less. By controlling the content of the saturated cyclic carbonate to be in this range, the resistance of the nonaqueous electrolyte solution to oxidation and reduction is improved, so that the stability during high-temperature storage tends to be improved.

It is noted here that, in the present invention, "% by volume" means a volume at 25°C and 1 atm.

<1-8-2. Linear Carbonate>

As the linear carbonate, one having 3 to 7 carbon atoms is usually used and, for the purpose of adjusting the viscosity of the electrolyte solution to be in an appropriate range, a linear carbonate having 3 to 5 carbon atoms is preferably used.

Specific examples of the linear carbonate include dimethyl carbonate, diethyl carbonate, di-*n*-propyl carbonate, diisopropyl carbonate, *n*-propyl isopropyl carbonate, ethyl methyl carbonate, methyl-n-propyl carbonate, *n*-butyl methyl carbonate, isobutyl methyl carbonate, *t*-butyl methyl carbonate, ethyl-n-propyl carbonate, *n*-butyl ethyl carbonate, isobutyl ethyl carbonate, and *t*-butyl ethyl carbonate.

Thereamong, dimethyl carbonate, diethyl carbonate, di-*n*-propyl carbonate, diisopropyl carbonate, *n*-propyl isopropyl carbonate, ethyl methyl carbonate and methyl-n-propyl carbonate are preferred, and dimethyl carbonate, diethyl carbonate and ethyl methyl carbonate are particularly preferred.

Further, a fluorine atom-containing linear carbonate (hereinafter, may be simply referred to as "fluorinated linear carbonate") can be preferably used as well. The number of fluorine atoms in the fluorinated linear carbonate is not particularly restricted as long as it is one or more; however, it is usually 6 or less, preferably 4 or less. When the fluorinated linear carbonate has plural fluorine atoms, the fluorine atoms may be bound to the same carbon, or may be bound to different carbons. Examples of the fluorinated linear carbonate include fluorinated dimethyl carbonate derivatives, fluorinated ethyl methyl carbonate derivatives, and fluorinated diethyl carbonate derivatives.

Examples of the fluorinated dimethyl carbonate derivatives include fluoromethyl methyl carbonate, difluoromethyl methyl carbonate, trifluoromethyl methyl carbonate, bis(fluoromethyl)carbonate, bis(difluoro)methyl carbonate, and bis(trifluoromethyl)carbonate.

Examples of the fluorinated ethyl methyl carbonate derivatives include 2-fluoroethyl methyl carbonate, ethyl fluoromethyl carbonate, 2,2-difluoroethyl methyl carbonate, 2-fluoroethyl fluoromethyl carbonate, ethyl difluoromethyl carbonate, 2,2,2-trifluoroethyl methyl carbonate, 2,2-difluoroethyl fluoromethyl carbonate, 2-fluoroethyl difluoromethyl carbonate, and ethyl trifluoromethyl carbonate.

Examples of the fluorinated diethyl carbonate derivatives include ethyl-(2-fluoroethyl)carbonate, ethyl-(2,2-difluoroethyl)carbonate, bis(2-fluoroethyl)carbonate, ethyl-(2,2,2-trifluoroethyl)carbonate, 2,2-difluoroethyl-2'-fluoroethyl carbonate, bis(2,2-difluoroethyl)carbonate, 2,2,2-trifluoroethyl-2'-fluoroethyl carbonate, 2,2,2-trifluoroethyl-2',2'-difluoroethyl carbonate, and bis(2,2,2-trifluoroethyl)carbonate.

Any of the above-described linear carbonates may be used singly, or two or more thereof may be used in any combination at any ratio.

The content of the linear carbonate is not particularly restricted; however, it is usually 15% by volume or higher, preferably 20% by volume or higher, more preferably 25% by volume or higher, but usually 90% by volume or less, preferably 85% by volume or less, more preferably 80% by volume or less, with respect to a total solvent amount of the nonaqueous electrolyte solution. By controlling the content of the linear carbonate to be in this range, the viscosity of the nonaqueous electrolyte solution is kept in an appropriate range and a reduction in the ionic conductivity is inhibited, as a result of which the output characteristics of a nonaqueous electrolyte battery are likely to be attained in a favorable range. When two or more linear carbonates are used in combination, a total amount thereof should satisfy the above-described range.

Moreover, the battery performance can be markedly improved by incorporating a specific amount of ethylene carbonate in combination with a specific linear carbonate.

For example, when dimethyl carbonate and ethyl methyl carbonate are selected as specific linear carbonates, the content of ethylene carbonate is not particularly restricted and may be set arbitrarily as long as the effects of the present invention are not markedly impaired; however, it is usually 15% by volume or higher, preferably 20% by volume or higher, but usually 45% by volume or less, preferably 40% by volume or less, with respect to a total solvent amount of the nonaqueous electrolyte solution; the content of dimethyl carbonate is usually 20% by volume or higher, preferably 30% by volume or higher, but usually 50% by volume or less, preferably 45% by volume or less, with respect to a total solvent amount of the nonaqueous electrolyte solution; and the content of ethyl methyl carbonate is usually 20% by

volume or higher, preferably 30% by volume or higher, but usually 50% by volume or less, preferably 45% by volume or less. By controlling these content values to be in the above-described respective ranges, excellent high-temperature stability is attained and gas generation tends to be inhibited.

<1-8-3. Ether-Based Compound>

The ether-based compound is preferably a linear ether having 3 to 10 carbon atoms, or a cyclic ether having 3 to 6 carbon atoms.

Examples of the linear ether having 3 to 10 carbon atoms include diethyl ether, di(2-fluoroethyl)ether, di(2,2-difluoroethyl)ether, di(2,2,2-trifluoroethyl)ether, ethyl(2-fluoroethyl)ether, ethyl(2,2,2-trifluoroethyl)ether, ethyl(1,1,2,2-tetrafluoroethyl)ether, (2-fluoroethyl)(2,2,2-trifluoroethyl)ether, (2-fluoroethyl)(1,1,2,2-tetrafluoroethyl)ether, (2,2,2-trifluoroethyl)(1,1,2,2-tetrafluoroethyl)ether, ethyl-n-propyl ether, ethyl(3-fluoro-*n*-propyl)ether, ethyl(3,3,3-trifluoro-*n*-propyl)ether, ethyl(2,2,3,3-tetrafluoro-*n*-propyl)ether, ethyl(2,2,3,3,3-pentafluoro-*n*-propyl)ether, 2-fluoroethyl-*n*-propyl ether, (2-fluoroethyl)(3-fluoro-*n*-propyl)ether, (2-fluoroethyl)(3,3,3-trifluoro-*n*-propyl)ether, (2-fluoroethyl)(2,2,3,3-tetrafluoro-*n*-propyl)ether, (2-fluoroethyl)(2,2,3,3,3-pentafluoro-*n*-propyl)ether, 2,2,2-trifluoroethyl-*n*-propyl ether, (2,2,2-trifluoroethyl)(3-fluoro-*n*-propyl)ether, (2,2,2-trifluoroethyl)(3,3,3-trifluoro-*n*-propyl)ether, (2,2,2-trifluoroethyl)(2,2,3,3-tetrafluoro-*n*-propyl)ether, (2,2,2-trifluoroethyl)(2,2,3,3,3-pentafluoro-*n*-propyl)ether, 1,1,2,2-tetrafluoroethyl-*n*-propyl ether, (1,1,2,2-tetrafluoroethyl)(3-fluoro-*n*-propyl)ether, (1,1,2,2-tetrafluoroethyl)(3,3,3 -trifluoro-*n*-propyl)ether, (1,1,2,2-tetrafluoroethyl)(2,2,3,3 -tetrafluoro-*n*-propyl)ether, (1,1,2,2-tetrafluoroethyl)(2,2,3,3,3-pentafluoro-*n*-propyl)ether, di-*n*-propyl ether, (*n*-propyl)(3-fluoro-*n*-propyl)ether, (*n*-propyl)(3,3,3-trifluoro-*n*-propyl)ether, (*n*-propyl)(2,2,3,3-tetrafluoro-*n*-propyl)ether, (*n*-propyl)(2,2,3,3,3-pentafluoro-*n*-propyl)ether, di(3-fluoro-*n*-propyl)ether, (3-fluoro-*n*-propyl)(3,3,3-trifluoro-*n*-propyl)ether, (3-fluoro-*n*-propyl)(2,2,3,3-tetrafluoro-*n*-propyl)ether, (3-fluoro-*n*-propyl)(2,2,3,3,3-pentafluoro-*n*-propyl)ether, di(3,3,3-trifluoro-*n*-propyl)ether, (3,3,3-trifluoro-*n*-propyl)(2,2,3,3-tetrafluoro-*n*-propyl)ether, (3,3,3-trifluoro-*n*-propyl)(2,2,3,3,3-pentafluoro-*n*-propyl)ether, di(2,2,3,3-tetrafluoro-*n*-propyl)ether, (2,2,3,3-tetrafluoro-*n*-propyl)(2,2,3,3,3-pentafluoro-*n*-propyl)ether, di(2,2,3,3,3-pentafluoro-*n*-propyl)ether, di-*n*-butyl ether, dimethoxymethane, ethoxymethoxymethane, methoxy(2-fluoroethoxy)methane, methoxy(2,2,2-trifluoroethoxy)methane, methoxy(1,1,2,2-tetrafluoroethoxy)methane, diethoxymethane, ethoxy(2-fluoroethoxy)methane, ethoxy(2,2,2-trifluoroethoxy)methane, ethoxy(1,1,2,2-tetrafluoroethoxy)methane, di(2-fluoroethoxy)methane, (2-fluoroethoxy)(2,2,2-trifluoroethoxy)methane, (2-fluoroethoxy)(1,1,2,2-tetrafluoroethoxy)methane, di(2,2,2-trifluoroethoxy)methane, (2,2,2-trifluoroethoxy)(1,1,2,2-tetrafluoroethoxy)methane, di(1,1,2,2-tetrafluoroethoxy)methane, dimethoxyethane, methoxyethoxyethane, methoxy(2-fluoroethoxy)ethane, methoxy(2,2,2-trifluoroethoxy)ethane, methoxy(1,1,2,2-tetrafluoroethoxy)ethane, diethoxyethane, ethoxy(2-fluoroethoxy)ethane, ethoxy(2,2,2-trifluoroethoxy)ethane, ethoxy(1,1,2,2-tetrafluoroethoxy)ethane, di(2-fluoroethoxy)ethane, (2-fluoroethoxy)(2,2,2-trifluoroethoxy)ethane, (2-fluoroethoxy)(1,1,2,2-tetrafluoroethoxy)ethane, di(2,2,2-trifluoroethoxy)ethane, (2,2,2-trifluoroethoxy)(1,1,2,2-tetrafluoroethoxy)ethane, di(1,1,2,2-tetrafluoroethoxy)ethane, ethylene glycol di-*n*-propyl ether, ethylene glycol di-*n*-butyl ether, and diethylene glycol dimethyl ether.

Examples of the cyclic ether having 3 to 6 carbon atoms include tetrahydrofuran, 2-methyltetrahydrofuran, 3-methyltetrahydrofuran, 1,3-dioxane, 2-methyl-1,3-dioxane, 4-methyl-1,3-dioxane, 1,4-dioxane, and fluorinated compounds thereof.

Thereamong, dimethoxymethane, diethoxymethane, ethoxymethoxymethane, ethylene glycol di-*n*-propyl ether, ethylene glycol di-*n*-butyl ether, and diethylene glycol dimethyl ether are preferred since they have a high solvating capacity with lithium ions and thus improve the ion dissociation. Particularly preferred are dimethoxymethane, diethoxymethane, and ethoxymethoxymethane since they have a low viscosity and provide a high ionic conductivity.

Any of the above-described ether-based compounds may be used singly, or two or more thereof may be used in any combination at any ratio.

The content of the ether-based compound is not particularly restricted and may be set arbitrarily as long as the effects of the present invention are not markedly impaired; however, it is usually 1% by volume or higher, preferably 2% by volume or higher, more preferably 3% by volume or higher, but usually 30% by volume or less, preferably 25% by volume or less, more preferably 20% by volume or less, in 100% by volume of the nonaqueous solvent. When two or more ether-based compounds are used in combination, a total amount thereof should satisfy the above-described range. When the content of the ether-based compound(s) is in this preferred range, an ionic conductivity-improving effect of a linear ether, which is attributed to an increase in the degree of lithium ion dissociation and a reduction in the viscosity, is likely to be ensured. In addition, when the negative electrode active material is a carbonaceous material, the phenomenon of co-intercalation of a linear ether thereto along with lithium ions can be inhibited; therefore, the input-output characteristics and the charge-discharge rate characteristics can be attained in appropriate ranges.

<1-8-4. Sulfone-Based Compound>

The sulfone-based compound is not particularly restricted regardless of whether it is a cyclic sulfone or a linear

sulfone. In the case of a cyclic sulfone, the number of its carbon atoms is usually 3 to 6, preferably 3 to 5, while in the case of a linear sulfone, the number of its carbon atoms is usually 2 to 6, preferably 2 to 5. The number of sulfonyl groups in one molecule of the sulfone-based compound is also not particularly restricted; however, it is usually 1 or 2.

Examples of the cyclic sulfone include: monosulfone compounds, such as trimethylene sulfones, tetramethylene sulfones, and hexamethylene sulfones; and disulfone compounds, such as trimethylene disulfones, tetramethylene disulfones, and hexamethylene disulfones. Thereamong, from the standpoints of the dielectric constant and the viscosity, tetramethylene sulfones, tetramethylene disulfones, hexamethylene sulfones and hexamethylene disulfones are more preferred, and tetramethylene sulfones (sulfolanes) are particularly preferred.

As the sulfolanes, sulfolane and sulfolane derivatives (hereinafter, may be simply referred to as "sulfolanes", including sulfolane) are preferred. As the sulfolane derivatives, those in which one or more hydrogen atoms bound to carbon atoms constituting a sulfolane ring are substituted with a fluorine atom or an alkyl group are preferred.

Among such sulfolane derivatives, for example, 2-methyl sulfolane, 3-methyl sulfolane, 2-fluorosulfolane, 3-fluorosulfolane, 2,2-difluorosulfolane, 2,3-difluorosulfolane, 2,4-difluorosulfolane, 2,5-difluorosulfolane, 3,4-difluorosulfolane, 2-fluoro-3-methyl sulfolane, 2-fluoro-2-methyl sulfolane, 3-fluoro-3-methyl sulfolane, 3-fluoro-2-methyl sulfolane, 4-fluoro-3-methyl sulfolane, 4-fluoro-2-methyl sulfolane, 5-fluoro-3-methyl sulfolane, 5-fluoro-2-methyl sulfolane, 2-fluoromethyl sulfolane, 3-fluoromethyl sulfolane, 2-difluoromethyl sulfolane, 3-difluoromethyl sulfolane, 2-trifluoromethyl sulfolane, 3-trifluoromethyl sulfolane, 2-fluoro-3-(trifluoromethyl)sulfolane, 3-fluoro-3-(trifluoromethyl)sulfolane, 4-fluoro-3-(trifluoromethyl)sulfolane, and 5-fluoro-3-(trifluoromethyl)sulfolane are preferred from the standpoint of attaining a high ionic conductivity and a high input/output.

Examples of the linear sulfone include dimethyl sulfone, ethyl methyl sulfone, diethyl sulfone, *n*-propyl methyl sulfone, *n*-propyl ethyl sulfone, di-*n*-propyl sulfone, isopropyl methyl sulfone, isopropyl ethyl sulfone, diisopropyl sulfone, *n*-butyl methyl sulfone, *n*-butyl ethyl sulfone, *t*-butyl methyl sulfone, *t*-butyl ethyl sulfone, monofluoromethyl methyl sulfone, difluoromethyl methyl sulfone, trifluoromethyl methyl sulfone, monofluoroethyl methyl sulfone, difluoroethyl methyl sulfone, trifluoroethyl methyl sulfone, pentafluoroethyl methyl sulfone, ethyl monofluoromethyl sulfone, ethyl difluoromethyl sulfone, ethyl trifluoromethyl sulfone, perfluoroethyl methyl sulfone, ethyl trifluoroethyl sulfone, ethyl pentafluoroethyl sulfone, di(trifluoroethyl)sulfone, perfluorodiethyl sulfone, fluoromethyl-*n*-propyl sulfone, difluoromethyl-*n*-propyl sulfone, trifluoromethyl-*n*-propyl sulfone, fluoromethyl isopropyl sulfone, difluoromethyl isopropyl sulfone, trifluoromethyl isopropyl sulfone, trifluoroethyl-n-propyl sulfone, trifluoroethyl isopropyl sulfone, pentafluoroethyl-n-propyl sulfone, pentafluoroethyl isopropyl sulfone, trifluoroethyl-*n*-butyl sulfone, trifluoroethyl-*t*-butyl sulfone, pentafluoroethyl-n-butyl sulfone, and pentafluoroethyl-*t*-butyl sulfone.

Thereamong, for example, dimethyl sulfone, ethyl methyl sulfone, diethyl sulfone, *n*-propyl methyl sulfone, isopropyl methyl sulfone, *n*-butyl methyl sulfone, *t*-butyl methyl sulfone, monofluoromethyl methyl sulfone, difluoromethyl methyl sulfone, trifluoromethyl methyl sulfone, monofluoroethyl methyl sulfone, difluoroethyl methyl sulfone, trifluoroethyl methyl sulfone, pentafluoroethyl methyl sulfone, ethyl monofluoromethyl sulfone, ethyl difluoromethyl sulfone, ethyl trifluoromethyl sulfone, ethyl trifluoroethyl sulfone, ethyl pentafluoroethyl sulfone, trifluoromethyl-*n*-propyl sulfone, trifluoromethyl isopropyl sulfone, trifluoroethyl-*n*-butyl sulfone, trifluoroethyl-*t*-butyl sulfone, trifluoromethyl-*n*-butyl sulfone, and trifluoromethyl-t-butyl sulfone are preferred from the standpoint of improving the high-temperature storage stability of the electrolyte solution.

Any of the above-described sulfone-based compounds may be used singly, or two or more thereof may be used in any combination at any ratio.

The content of the sulfone-based compound is not particularly restricted and may be set arbitrarily as long as the effects of the present invention are not markedly impaired; however, it is usually 0.3% by volume or higher, preferably 0.5% by volume or higher, more preferably 1% by volume or higher, but usually 40% by volume or less, preferably 35% by volume or less, more preferably 30% by volume or less, with respect to a total solvent amount of the nonaqueous electrolyte solution. When two or more sulfone-based compounds are used in combination, a total amount thereof should satisfy the above-described range. When the content of the sulfone-based compound(s) is in this range, an electrolyte solution having excellent high-temperature storage stability tends to be obtained.

<1-8. Auxiliary Agents>

The nonaqueous electrolyte solution of the present invention may also contain the following auxiliary agent(s) within a range that allows the effects of the present invention to be exerted.

Examples of the auxiliary agents include:

carbonate compounds, such as erythritan carbonate, spiro-bis-dimethylene carbonate, and methoxyethyl methyl carbonate;

triple bond-containing compounds, such as methyl-2-propynyl oxalate, ethyl-2-propynyl oxalate, bis(2-propynyl)oxalate, 2-propynyl acetate, 2-propynyl formate, 2-propynyl methacrylate, di(2-propynyl)glutarate, methyl-2-propynyl carbonate, ethyl-2-propynyl carbonate, bis(2-propynyl)carbonate, 2-butyne-1,4-diyl-dimethane sulfonate, 2-butyne-1,4-diyl-diethane sulfonate, 2-butyne-1,4-diyl-diformate, 2-butyne-1,4-diyl-diacetate, 2-butyne-1,4-diyl-dipropionate, 4-hexadiyne-1,6-diyl-dimethane sulfonate, 2-propynyl-methane sulfonate, 1-methyl-2-propynyl-methane sulfonate, 1,1-dimethyl-2-propynyl-methane sulfonate, 2-propynyl-ethane sulfonate, 2-propynyl-vinyl sulfonate, 2-propynyl-2-(diethoxyphosphoryl)acetate, 1-methyl-2-propynyl-2-(diethoxyphosphoryl)acetate, and 1,1-dimethyl-2-propynyl-2-(diethoxyphosphoryl)acetate;

spiro compounds, such as 2,4,8,10-tetraoxaspiro[5.5]undecane and 3,9-divinyl-2,4,8,10-tetraoxaspiro[5.5]undecane;

sulfur-containing compounds, such as ethylene sulfite, methyl fluorosulfonate, ethyl fluorosulfonate, methyl methanesulfonate, ethyl methanesulfonate, busulfan, sulfolene, ethylene sulfate, vinylene sulfate, diphenyl sulfone, *N,N*-dimethylmethane sulfonamide, *N,N*-diethylmethane sulfonamide, trimethylsilyl methyl sulfate, trimethylsilyl ethyl sulfate, and 2-propynyl-trimethylsilyl sulfate;

isocyanate compounds, such as 2-isocyanatoethyl acrylate, 2-isocyanatoethyl methacrylate, 2-isocyanatoethyl crotonate, 2-(2-isocyanatoethoxy)ethyl acrylate, 2-(2-isocyanatoethoxy)ethyl methacrylate, and 2-(2-isocyanatoethoxy)ethyl crotonate;

nitrogen-containing compounds, such as 1-methyl-2-pyrrolidinone, 1-methyl-2-piperidone, 3-methyl-2-oxazolidinone, 1,3-dimethyl-2-imidazolidinone, and *N*-methylsuccinimide;

hydrocarbon compounds, such as heptane, octane, nonane, decane, and cycloheptane;

fluorine-containing aromatic compounds, such as fluorobenzene, difluorobenzene, hexafluorobenzene, benzotrifluoride, o-fluorotoluene, *m*-fluorotoluene, *p*-fluorotoluene, 1,2-bis(trifluoromethyl)benzene, 1-trifluoromethyl-2-difluoromethylbenzene, 1,3-bis(trifluoromethyl)benzene, 1-trifluoromethyl-3-difluoromethylbenzene, 1,4-bis(trifluoromethyl)benzene, 1-trifluoromethyl-4-difluoromethylbenzene, 1,3,5-tris(trifluoromethyl)benzene, pentafluorophenylmethane sulfonate, pentafluorophenyltrifluoromethane sulfonate, pentafluorophenyl acetate, pentafluorophenyl trifluoroacetate, and methylpentafluorophenyl carbonate;

silane compounds, such as tris(trimethylsilyl)borate, tris(trimethoxysilyl)borate, tris(trimethylsilyl)phosphate, tris(trimethoxysilyl)phosphate, dimethoxyaluminoxytrimethoxysilane, diethoxyaluminoxytriethoxysilane, dipropoxyaluminoxytriethoxysilane, dibutoxyaluminoxytrimethoxysilane, dibutoxyaluminoxytriethoxysilane, titanium tetrakis(trimethylsiloxide), and titanium tetrakis(triethylsiloxide);

ester compounds, such as 2-propynyl 2-(methanesulfonyloxy)propionate, 2-methyl 2-(methanesulfonyloxy)propionate, 2-ethyl 2-(methanesulfonyloxy)propionate, 2-propynyl methanesulfonyloxyacetate, 2-methyl methanesulfonyloxyacetate, and 2-ethyl methanesulfonyloxyacetate; and

lithium salts, such as lithium ethylmethyloxycarbonyl phosphonate, lithium ethylethyloxycarbonyl phosphonate, lithium ethyl-2-propynyloxycarbonyl phosphonate, lithium ethyl-1-methyl-2-propynyloxycarbonyl phosphonate, and lithium ethyl-1,1-dimethyl-2-propynyloxycarbonyl phosphonate.

These auxiliary agents may be used singly, or in combination of two or more thereof. By adding these auxiliary agents, the capacity retention characteristics after high-temperature storage and the cycle characteristics can be improved.

The content of the auxiliary agent is not particularly restricted and may be set arbitrarily as long as the effects of the present invention are not markedly impaired. The content of the auxiliary agent is usually 0.01% by mass or higher, preferably 0.1% by mass or higher, more preferably 0.2% by mass or higher, but usually 5% by mass or less, preferably 3% by mass or less, more preferably 1% by mass or less, with respect to a total amount of the nonaqueous electrolyte solution. When the content of the auxiliary agent is in this range, the effects of the auxiliary agent are likely to be expressed sufficiently, so that the high-temperature storage stability tends to be improved. When two or more auxiliary agents are used in combination, a total amount thereof should satisfy the above-described range.

<2. Nonaqueous Electrolyte Battery>

The nonaqueous electrolyte battery of the present invention includes: a positive electrode that has a current collector and a positive electrode active material layer arranged on the current collector; a negative electrode that has a current collector and a negative electrode active material layer arranged on the current collector and is capable of absorbing and releasing metal ions; and the above-described nonaqueous electrolyte solution of the present invention.

<2-1. Battery Configuration>

The nonaqueous electrolyte battery of the present invention has the same configuration as that of a conventionally known nonaqueous electrolyte battery, except for the above-described nonaqueous electrolyte solution of the present invention. The nonaqueous electrolyte battery usually has a form in which the positive electrode and the negative

electrode are laminated via a porous membrane (separator) impregnated with the nonaqueous electrolyte solution of the present invention, and these components are housed in a casing (outer package). Accordingly, the shape of the nonaqueous electrolyte battery of the present invention is not particularly restricted and may be any of, for example, a cylindrical shape, a prismatic shape, a laminated shape, a coin shape, and a large-sized shape.

<2-2. Nonaqueous Electrolyte Solution>

As the nonaqueous electrolyte solution, the above-described nonaqueous electrolyte solution of the present invention is used. It is noted here that the above-described nonaqueous electrolyte solution of the present invention can also be blended with other nonaqueous electrolyte solution within a range that does not depart from the gist of the present invention.

<2-3. Negative Electrode>

A negative electrode active material used in the negative electrode will now be described. The negative electrode active material is not particularly restricted as long as it is capable of electrochemically absorbing and releasing metal ions. Specific examples thereof include: materials containing carbon as a constituent element, such as carbonaceous materials; and alloy-based materials. Any of these materials may be used singly, or two or more thereof may be used in any combination.

<2-3-1. Negative Electrode Active Substance>

As described above, the negative electrode active material is, for example, a carbonaceous material or an alloy-based material.

Examples of the carbonaceous material include (1) natural graphite, (2) artificial graphite, (3) amorphous carbon, (4) carbon-coated graphite, (5) graphite-coated graphite, and (6) resin-coated graphite.

Examples of the (1) natural graphite include scaly graphite, flaky graphite, earthy graphite, and graphite particles obtained by performing a treatment, such as spheronization or densification, on any of these graphites as a raw material. Thereamong, a spherical or ellipsoidal graphite obtained by a spheronization treatment is particularly preferred from the viewpoints of the packing property of its particles and the charge-discharge rate characteristics.

For the spheronization treatment, for example, an apparatus that repeatedly applies mechanical actions, such as compression, friction, shearing force and the like including particle interactions, mainly through impact force to particles can be used.

Specifically, it is preferred to use an apparatus which is equipped with a rotor having a large number of blades inside a casing and performs a spheronization treatment by rotating the rotor at a high speed and thereby applying mechanical actions, such as impact compression, friction and shearing force, to the natural graphite (1) as a raw material introduced to the inside. An apparatus that has a mechanism for repeatedly applying mechanical actions by circulation of the raw material is also preferred.

For example, when a spheronization treatment is performed using the above-described apparatus, the peripheral speed of the rotating rotor is set at preferably 30 to 100 m/sec, more preferably 40 to 100 m/sec, still more preferably 50 to 100 m/sec. The spheronization treatment can be performed by simply bringing the raw material through the apparatus; however, it is preferred to perform the treatment by circulating or retaining the raw material in the apparatus for at least 30 seconds, and it is more preferred to perform the treatment by circulating or retaining the raw material in the apparatus for 1 minute or longer.

Examples of the (2) artificial graphite include those produced by graphitizing an organic compound, such as coal-tar pitch, a coal-based heavy oil, an atmospheric residue oil, a petroleum-based heavy oil, an aromatic hydrocarbon, a nitrogen-containing cyclic compound, a sulfur-containing cyclic compound, a polyphenylene, a polyvinyl chloride, a polyvinyl alcohol, a polyacrylonitrile, a polyvinyl butyral, a natural polymer, a polyphenylene sulfide, a polyphenylene oxide, a furfuryl alcohol resin, a phenol-formaldehyde resin or an imide resin, at a temperature in a range of usually 2,500°C to 3,200°C, followed by pulverization and/or classification as required.

In this process, a silicon-containing compound, a boron-containing compound or the like can be used as a graphitization catalyst. Examples of the (2) artificial graphite also include those obtained by graphitizing mesocarbon microbeads separated in a pitch heat treatment process. Another example is artificial graphite of granulated particles composed of primary particles. Examples of such artificial graphite include graphite particles in which plural flat particles are aggregated or bound with each other such that their orientation planes are not parallel, which flat particles are obtained by, for example, mixing graphitizable carbonaceous material powder (e.g., mesocarbon microbeads or coke powder) with a graphitizable binder (e.g., tar or pitch) and a graphitization catalyst to perform graphitization, followed by pulverization of the resultant as required.

Examples of the (3) amorphous carbon include amorphous carbon particles obtained by heat-treating an easily graphitizable carbon precursor used as a raw material, such as tar or pitch, at least once in a temperature range where

graphitization does not occur (in a range of 400 to 2,200°C); and amorphous carbon particles obtained by heat-treating a hardly graphitizable carbon precursor used as a raw material, such as a resin.

Examples of the (4) carbon-coated graphite include those obtained in the following manner. A natural graphite and/or an artificial graphite is/are mixed with a carbon precursor, which is an organic compound such as tar, pitch or resin, and the resulting mixture is heat-treated at least once in a range of 400 to 2,300°C. Using this natural graphite and/or artificial graphite as core graphite, a carbon-graphite composite is obtained by coating the core graphite with amorphous carbon. This carbon-graphite composite is exemplified as the (4) carbon-coated graphite.

The above-described composite may take a form in which the surface of the core graphite is entirely or partially coated with amorphous carbon, or a form in which plural primary particles are combined using carbon derived from the above-described carbon precursor as a binder. Alternatively, the carbon-graphite composite can be obtained by allowing a natural graphite and/or an artificial graphite to react with a hydrocarbon gas, such as benzene, toluene, methane, propane, or an aromatic volatile component, at a high temperature and thereby depositing carbon on the graphite surface (CVD).

Examples of the (5) graphite-coated graphite include those obtained in the following manner. A natural graphite and/or an artificial graphite is/are mixed with a carbon precursor, which is an easily graphitizable organic compound such as tar, pitch or resin, and the resulting mixture is heat-treated at least once in a range of 2,400 to 3,200°C. Using this natural graphite and/or artificial graphite as core graphite, the (5) graphite-coated graphite is obtained by entirely or partially coating the surface of the core graphite with a graphitized product.

The (6) resin-coated graphite is obtained by, for example, mixing a natural graphite and/or an artificial graphite with a resin or the like, drying the resulting mixture at a temperature of lower than 400°C, and the coating the thus obtained core graphite with a resin or the like.

Any of the above-described carbonaceous materials of (1) to (6) may be used singly, or two or more thereof may be used in any combination at any ratio.

The organic compound such as tar, pitch or resin that is used in the above-described carbonaceous materials of (2) to (5) is, for example, a carbonizable organic compound selected from the group consisting of coal-based heavy oils, straight-run heavy oils, cracked petroleum heavy oils, aromatic hydrocarbons, N-ring compounds, S-ring compounds, polyphenylenes, organic synthetic polymers, natural polymers, thermoplastic resins, and thermosetting resins. In order to adjust the viscosity in mixing, the raw material organic compound may be used in the form of being dissolved in a low-molecular-weight organic solvent.

As the natural graphite and/or artificial graphite used as a raw material of the core graphite, a natural graphite which has been subjected to a spheronization treatment is preferred.

The above-described alloy-based material used as the negative electrode active material is not particularly restricted as long as it is capable of absorbing and releasing lithium ions, and may be lithium, a single substance metal or an alloy that forms an alloy with lithium, or any compound thereof such as an oxide, a carbide, a nitride, a silicide, a sulfide, or a phosphide. The metal or alloy that forms an alloy with lithium is:

preferably a material containing a metal or metalloid element of the periodic table Group 13 or 14 (i.e. excluding carbon),
more preferably a single substance metal of aluminum, silicon or tin, or an alloy or a compound that contains these atoms,
still more preferably a material containing silicon or tin as a constituent element, such as a single substance metal of silicon or tin, or an alloy or a compound that contains these atoms.

Any of these materials may be used singly, or two or more thereof may be used in any combination at any ratio.

<Metal Particles Alloyable with Li>

When a metal or an alloy that forms an alloy with lithium, or any compound thereof such as an oxide, a carbide, a nitride, a silicide, a sulfide, or a phosphide is used as the negative electrode active material, the metal alloyable with Li is in the form of particles. Examples of a method for confirming that the metal particles are alloyable with Li include identification of a metal particle phase by X-ray diffractometry, a combination of observation of the particle structure under an electron microscope and elemental analysis, and elemental analysis with fluorescent X-ray.

As the metal particles alloyable with Li, any conventionally known such metal particles can be used; however, from the standpoints of the capacity and the cycle life of the nonaqueous electrolyte battery, the metal particles are preferably particles of, for example, a metal selected from the group consisting of Fe, Co, Sb, Bi, Pb, Ni, Ag, Si, Sn, Al, Zr, Cr, P, S, V, Mn, As, Nb, Mo, Cu, Zn, Ge, In, Ti and W, or a compound thereof. Further, an alloy composed of two or more metals may be used as well, and the metal particles may be alloy particles formed by two or more metal elements. Thereamong, a metal selected from the group consisting of Si, Sn, As, Sb, Al, Zn and W, or a metal compound thereof

is preferred.

Examples of the metal compound include metal oxides, metal nitrides, and metal carbides. An alloy composed of two or more metals may be used as well.

Among these metal particles alloyable with Li, Si or an Si metal compound is preferred. The Si metal compound is preferably Si metal oxide. Si or the Si metal compound is preferred from the standpoint of increasing the battery capacity. In the present specification, Si and Si compounds are collectively referred to as "Si compounds". Specific examples of an Si compound include $SiO_x$, $SiN_x$, $SiC_x$, and $SiZ_xO_y$ (wherein, Z = C or N). The Si compound is preferably Si metal oxide which is represented by a general formula $SiO_x$. A compound represented by this general formula $SiO_x$ is obtained using silicon dioxide ($SiO_2$) and metal silicon (Si) as raw materials, and the value of x is usually $0 \leq x < 2$. $SiO_x$ has a higher theoretical capacity than graphite, and amorphous Si or nano-sized Si crystals facilitate migration of alkali ions such as lithium ions, so that a high capacity can be attained.

Specifically, an Si metal oxide is represented by $SiO_x$, wherein x is $0 \leq x < 2$, more preferably 0.2 to 1.8, still more preferably 0.4 to 1.6, particularly preferably 0.6 to 1.4. When x is in this range, the battery has a high capacity and, at the same time, the irreversible capacity attributed to binding between Li and oxygen can be reduced.

• Oxygen Content of Metal Particles Alloyable with Li

The oxygen content of the metal particles alloyable with Li is not particularly restricted; however, it is usually 0.01% by mass or higher and 8% by mass or less, preferably 0.05% by mass or higher and 5% by mass or less. As for the distribution state of oxygen in the particles, oxygen may exist near the surface or in the interior of the particles, or may uniformly exist throughout the particles; however, it is particularly preferred that oxygen exist near the surface. When the oxygen content of the metal particles alloyable with Li is in the above-described range, an increase in the volume caused by charging and discharging of a nonaqueous electrolyte secondary battery is suppressed because of strong binding between the metal particles and O (oxygen atoms), so that excellent cycle characteristics are attained, which is preferred.

<Negative Electrode Active Material That Contains Metal Particles Alloyable with Li and Graphite Particles>

The negative electrode active material may contain metal particles alloyable with Li and graphite particles. The negative electrode active material may be a mixture in which the metal particles alloyable with Li and the graphite particles are mixed in a state of mutually independent particles, or may be a composite in which the metal particles alloyable with Li exist on the surface of the graphite particles or inside the graphite particles.

The composite of the metal particles alloyable with Li and graphite particles (hereinafter, also referred to as "composite particles") is not particularly restricted as long as it contains the metal particles alloyable with Li and graphite particles; however, the composite particles are preferably particles in which the metal particles alloyable with Li and the graphite particles are integrated together by physical and/or chemical bonds. In a more preferred mode, the metal particles alloyable with Li and the graphite particles are in a state where their solid components are dispersed in the composite particles to such an extent that allows them to exist at least both on the surface of the composite particles and inside the bulk, and the graphite particles exist in a manner to integrate the solid components via physical and/or chemical bonds. In a still more preferred form, the composite is a composite material (negative electrode active material) that is composed of at least metal particles alloyable with Li and graphite particles, in which inside of the graphite particles, preferably particles of natural graphite having a folded structure with a curved surface, the metal particles alloyable with Li exist in the gaps in the structure. The gaps may be voids, and a substance that mitigates expansion and contraction of the metal particles alloyable with Li, such as amorphous carbon, a graphitic material or a resin, may exist in the gaps. Particles of natural graphite with a curved structure and a folded structure

• Content Ratio of Metal Particles Alloyable with Li

The content ratio of the metal particles alloyable with Li is usually 0.1% by mass or higher, preferably 0.5% by mass or higher, more preferably 1.0% by mass or higher, still more preferably 2.0% by mass or higher, but usually 99% by mass or lower, preferably 50% by mass or lower, more preferably 40% by mass or lower, still more preferably 30% by mass or lower, yet still more preferably 25% by mass or lower, yet still more preferably 20% by mass or lower, particularly preferably 15% by mass or lower, most preferably 10% by mass or lower, with respect to a total amount of the metal particles alloyable with Li and the graphite particles. When the content ratio of the metal particles alloyable with Li is in this range, side reactions on the Si surface can be controlled, so that the nonaqueous electrolyte battery can attain a sufficient capacity, which is preferred.

(Coverage)

The negative electrode active material of the present invention may be covered with a carbonaceous material or a graphitic material. Particularly, the negative electrode active material of the present invention is preferably covered with an amorphous carbonaceous material from the standpoint of the lithium ion acceptability. The coverage is usually 0.5% to 30%, preferably 1% to 25%, more preferably 2% to 20%. It is preferred that the upper limit of the coverage be in this range from the standpoint of the reversible capacity when the negative electrode active material is incorporated into a battery, while the lower limit of the coverage be in this range from the standpoint of allowing the carbonaceous material serving as a core to be uniformly coated with amorphous carbon and thereby achieving strong granulation as well as from the standpoint of the size of the particles obtained by post-firing pulverization.

The coverage (content ratio) of a carbide derived from an organic compound of the ultimately obtained negative electrode active material can be calculated by the following formula based on the amount of the negative electrode active material, the amount of the organic compound, and the residual carbon ratio determined by the micro method according to JIS K2270.

$$\text{Coverage (\%) of carbide derived from organic compound} = (\text{Mass of organic compound} \times \text{Residual carbon ratio} \times 100)/\{\text{Mass of negative electrode active material} + (\text{Mass of organic compound} \times \text{Residual carbon ratio})\}$$

(Internal Porosity)

The internal porosity of the negative electrode active material is usually 1% or higher, preferably 3% or higher, more preferably 5% or higher, still more preferably 7% or higher, but usually lower than 50%, preferably 40% or lower, more preferably 30% or lower, still more preferably 20% or lower. When the internal porosity is excessively low, the liquid amount inside the particles of the negative electrode active material in a nonaqueous electrolyte battery tends to be small. Meanwhile, with the internal porosity being excessively high, the amount of gaps between the particles tends to be small when the negative electrode active material is used in an electrode. It is preferred that the lower limit of the internal porosity be in the above-described range from the standpoint of the charge-discharge characteristics, while the upper limit of the internal porosity be in the above-described range from the standpoint of dispersion of the nonaqueous electrolyte solution. Further, as described above, the gaps may be voids, and a substance that mitigates expansion and contraction of the metal particles alloyable with Li, such as amorphous carbon, a graphitic material or a resin, may exist in the gaps, or the gaps may be filled with such a substance.

<2-3-2. Constitution and Production Method of Negative Electrode>

For the production of the negative electrode, any known method can be employed as long as it does not markedly limit the effects of the present invention. For example, a binder, a solvent and, as required, a thickening agent, a conductive material, a filler and the like are added to the negative electrode active material to prepare a slurry, and this slurry is subsequently coated and dried onto a current collector, followed by pressing of the resultant, whereby the negative electrode can be formed.

Further, a negative electrode of an alloy-based material can be produced by any known method. Specifically, examples of a method of producing the negative electrode include: a method of producing a sheet electrode by adding a binder, a conductive material and the like to the above-described negative electrode active material and then directly roll-molding the resulting mixture; and a method of producing a pellet electrode by compression-molding the mixture; however, a method of forming a thin film layer containing the above-described negative electrode active material (negative electrode active material layer) on a current collector for negative electrode (hereinafter, may be referred to as "negative electrode current collector") by means of coating, vapor deposition, sputtering, plating or the like is usually employed. In this case, a binder, a thickening agent, a conductive material, a solvent and the like are added to the above-described negative electrode active material to prepare a slurry, and this slurry is coated and dried onto a negative electrode current collector, after which the resultant is pressed to increase the density, whereby a negative electrode active material layer is formed on the negative electrode current collector.

Examples of the material of the negative electrode current collector include steel, copper, copper alloys, nickel, nickel alloys, and stainless steel. Thereamong, a copper foil is preferred from the standpoints of the ease of processing into a thin film and the cost.

The thickness of the negative electrode current collector is usually 1 μm or greater, preferably 5 μm or greater, but usually 100 μm or less, preferably 50 μm or less. This is because an overly thick negative electrode current collector may cause an excessive reduction in the capacity of the whole nonaqueous electrolyte battery, while an overly thin negative electrode current collector may be difficult to handle.

The surface of the negative electrode current collector is preferably subjected to a roughening treatment in advance for the purpose of improving its binding with the negative electrode active material layer formed thereon. Examples of a surface roughening method include: blasting; rolling with a surface-roughened roll; mechanical polishing in which the current collector surface is polished with a polishing cloth or paper on which abrasive particles are fixed, a whetstone, an emery buff, or a wire brush equipped with steel wires or the like; electrolytic polishing; and chemical polishing.

Alternatively, in order to reduce the mass of the negative electrode current collector and to thereby increase the energy density of a battery per unit mass, a perforated negative electrode current collector in the form of an expanded metal or a punched metal can be used as well. In the negative electrode current collector of this type, the mass can be modified as desired by changing the opening ratio. In addition, when a negative electrode active material layer is formed on both sides of the negative electrode current collector of this type, a riveting effect through perforations makes the negative electrode active material layer less likely to be detached. However, an excessively high opening ratio rather reduces the adhesive strength due to a small contact surface area between the negative electrode active material layer and the negative electrode current collector.

The slurry used for the formation of the negative electrode active material layer is usually prepared by adding a binder, a thickening agent and the like to a negative electrode material. It is noted here that the term "negative electrode material" used herein refers to a material obtained by combining a negative electrode active material and a conductive material.

In the negative electrode material, the content of the negative electrode active material is usually 70% by mass or higher, particularly 75% by mass or higher, but usually 97% by mass or less, particularly preferably 95% by mass or less. When the content of the negative electrode active material is excessively low, the capacity of a secondary battery using the resulting negative electrode tends to be insufficient, whereas when the content of the negative electrode active material is excessively high, a relatively insufficient amount of the conductive material tends to make it difficult to ensure the resulting negative electrode to have an adequate electrical conductivity. In the case of using two or more negative electrode active materials in combination, a total amount of the negative electrode active materials should satisfy the above-described range.

Examples of the conductive material used in the negative electrode include metal materials, such as copper and nickel; and carbon materials, such as graphite and carbon black. Any of these conductive materials may be used singly, or two or more thereof may be used in any combination at any ratio. Particularly, it is preferred to use a carbon material as the conductive material since the carbon material also acts as an active material. The content of the conductive material in the negative electrode material is usually 3% by mass or higher, particularly preferably 5% by mass or higher, but usually 30% by mass or less, preferably 25% by mass or less. An excessively low content of the conductive material tends to make the conductivity insufficient, while an excessively high content of the conductive material tends to result in a reduction in the battery capacity and strength due to a relatively insufficient amount of the negative electrode active material and the like. In the case of using two or more conductive materials in combination, a total amount of the conductive materials should satisfy the above-described range.

The binder used in the negative electrode may be any binder as long as it is a material that is stable against the solvent and the electrolyte solution that are used in the electrode production. Examples of the binder include polyvinylidene fluoride, polytetrafluoroethylene, polyethylene, polypropylene, styrene-butadiene rubber, isoprene rubber, butadiene rubber, ethylene-acrylic acid copolymers, and ethylene-methacrylic acid copolymers. Any of these binders may be used singly, or two or more thereof may be used in any combination at any ratio. The content of the binder is usually 0.5 parts by mass or higher, preferably 1 part by mas or higher, but usually 10 parts by mass or less, preferably 8 parts by mass or less, with respect to 100 parts by mass of the negative electrode material. When the content of the binder is excessively low, the strength of the resulting negative electrode tends to be insufficient, whereas when the content of the binder is excessively high, the battery capacity and conductivity tend to be insufficient due to a relatively insufficient amount of the negative electrode active material and the like. In the case of using two or more binders in combination, a total amount of the binders should satisfy the above-described range.

Examples of the thickening agent used in the negative electrode include carboxymethyl cellulose, methyl cellulose, hydroxymethyl cellulose, ethyl cellulose, polyvinyl alcohol, oxidized starch, phosphorylated starch, and casein. Any of these thickening agents may be used singly, or two or more thereof may be used in any combination at any ratio. The thickening agent may be used as required; however, when used, usually, the content thereof in the negative electrode active material layer is preferably in a range of 0.5% by mass or higher and 5% by mass or less.

The slurry used for the formation of the negative electrode active material layer is prepared by mixing the above-described negative electrode active material with a conductive material, a binder and a thickening agent as required,

using an aqueous solvent or an organic solvent as a dispersion medium. Water is usually used as the aqueous solvent; however, an organic solvent, for example, an alcohol such as ethanol or a cyclic amide such as *N*-methylpyrrolidone, may also be used in combination in a range of 30% by mass or less with respect to water. Examples of the organic solvent usually include: cyclic amides, such as *N*-methylpyrrolidone; straight-chain amides, such as *N*,*N*-dimethylformamide and *N*,*N*-dimethylacetamide; aromatic hydrocarbons, such as anisole, toluene, and xylene; and alcohols, such as butanol and cyclohexanol, among which cyclic amides such as *N*-methylpyrrolidone, and straight-chain amides such as *N*,*N*-dimethylformamide and N,N-dimethylacetamide are preferred. Any of these organic solvents may be used singly, or two or more thereof may be used in any combination at any ratio.

The resulting slurry is coated and dried onto the above-described negative electrode current collector, and the resultant is subsequently pressed to form a negative electrode active material layer, whereby a negative electrode is obtained. A coating method is not particularly restricted, and any known method can be employed. A drying method is also not particularly restricted, and any known method such as air drying, heat drying, or vacuum drying can be employed.

(Electrode Density)

The structure of an electrode formed from the negative electrode active material is not particularly restricted, and the density of the negative electrode active material existing on the current collector is preferably 1 $g \cdot cm^{-3}$ or higher, more preferably 1.2 $g \cdot cm^{-3}$ or higher, still more preferably 1.3 $g \cdot cm^{-3}$ or higher, but preferably 2.2 $g \cdot cm^{-3}$ or lower, more preferably 2.1 $g \cdot cm^{-3}$ or lower, still more preferably 2.0 $g \cdot cm^{-3}$ or lower, particularly preferably 1.9 $g \cdot cm^{-3}$ or lower. When the density of the negative electrode active material existing on the current collector is higher than this range, particles of the negative electrode active material may be destructed to cause an increase in the initial irreversible capacity of a nonaqueous electrolyte battery and a reduction in the permeability of the nonaqueous electrolyte solution to the vicinity of the interface between the current collector and the negative electrode active material, as a result of which the high-current-density charge-discharge characteristics may be deteriorated. Meanwhile, when the density of the negative electrode active material is lower than the above-described range, the conductivity between the negative electrode active material is reduced and the battery resistance is thus increased, as a result of which the capacity per unit volume may be reduced.

<2-4. Positive Electrode>

The positive electrode used in the nonaqueous electrolyte battery of the present invention will now be described.

<2-4-1. Positive Electrode Active Substance>

First, a positive electrode active material used in the positive electrode will be described.

(1) Composition

The positive electrode active material is not particularly restricted as long as it is lithium cobaltate, or a transition metal oxide containing at least Ni and Co in which Ni and Co constitute not less than 50% by mole of transition metals and which is capable of electrochemically absorbing and releasing metal ions, and the positive electrode active material is preferably, for example, a transition metal oxide which is capable of electrochemically absorbing and releasing lithium ions and contains lithium along with at least Ni and Co and in which Ni and Co constitute not less than 50% by mole of transition metals. Ni and Co have a redox potential suitable for the use as positive electrode materials of a secondary battery, and are thus appropriate for high-capacity applications.

As transition metal components of such a lithium transition metal oxide, Ni and Co are contained as indispensable elements, and examples of other metals include Mn, V, Ti, Cr, Fe, Cu, Al, Mg, Zr and Er, among which, for example, Mn, Ti, Fe, Al, Mg and Zr are preferred. Specific examples of the lithium transition metal oxide include $LiCoO_2$, $LiNi_{0.85}Co_{0.10}Al_{0.05}O_2$, $LiNi_{0.80}Co_{0.15}Al_{0.05}O_2$, $LiNi_{0.33}CO_{0.33}Mn_{0.33}O_2$, $Li_{1.05}Ni_{0.33}Mn_{0.33}CO_{0.33}O_2$, $LiNi_{0.5}Co_{0.2}Mn_{0.3}O_2$, $Li_{1.05}Ni_{0.50}Mn_{0.29}CO_{0.21}O_2$, $LiNi_{0.6}Co_{0.2}Mn_{0.2}O_2$, and $LiNi_{0.8}Co_{0.1}Mn_{0.1}O_2$.

Thereamong, the lithium transition metal oxide is preferably a transition metal oxide represented by the following composition formula (1):

$$Li_{a1}Ni_{b1}Co_{c1}M_{d1}O_2 \qquad (1)$$

(wherein, a1, b1, c1 and d1 represent numerical values of $0.90 \leq a1 \leq 1.10$, $0.50 \leq b1 \leq 0.98$, $0.01 \leq c1 < 0.50$ and $0.01 \leq d1 < 0.50$, satisfying $b1 + c1 + d1 = 1$; and M represents at least one element selected from the group consisting of Mn, Al, Mg, Zr, Fe, Ti, and Er).

In the composition formula (1), d1 preferably represents a numerical value of $0.1 \leq d1 < 0.5$.

By controlling the composition ratios of Ni, Co and other metal species as prescribed above, the transition metals are made unlikely to elute out of the positive electrode and, even if they did, Ni and Co would have only a small adverse effect in the nonaqueous secondary battery.

The lithium transition metal oxide is more preferably a transition metal oxide represented by the following composition formula (2):

$$Li_{a2}Ni_{b2}CO_{c2}M_{d2}O_2 \qquad (2)$$

(wherein, a2, b2, c2 and d2 represent numerical values of $0.90 \leq a2 \leq 1.10$, $0.50 \leq b2 \leq 0.96$, $0.03 \leq c2 < 0.50$ and $0.01 \leq d2 < 0.40$, satisfying $b2 + c2 + d2 = 1$; and M represents at least one element selected from the group consisting of Mn, Al, Mg, Zr, Fe, Ti, and Er).

In the composition formula (2), d2 preferably represents a numerical value of $0.10 \leq d2 < 0.40$.

By allowing the lithium transition metal oxide to contain Ni and Co as main components and controlling the composition ratio of Ni to be higher than that of Co, a good stability can be attained and a high capacity can be extracted when the lithium transition metal oxide is used as the positive electrode of the nonaqueous battery.

Especially, the lithium transition metal oxide is still more preferably a transition metal oxide represented by the following composition formula (3):

$$Li_{a3}Ni_{b3}CO_{c3}M_{d3}O_2 \qquad (3)$$

(wherein, a3, b3, c3 and d3 represent numerical values of $0.90 \leq a3 \leq 1.10$, $0.60 \leq b3 \leq 0.94$, $0.05 \leq c3 \leq 0.2$ and $0.01 \leq d3 \leq 0.3$, satisfying $b3 + c3 + d3 = 1$; and M represents at least one element selected from the group consisting of Mn, Al, Mg, Zr, Fe, Ti, and Er).

In the composition formula (3), d3 preferably represents a numerical value of $0.10 \leq d3 \leq 0.3$.

By allowing the lithium transition metal oxide to have the above-described composition, a particularly high capacity can be extracted when it is used as the positive electrode of the nonaqueous secondary battery.

Further, two or more of the above-described positive electrode active materials may be used as a mixture. Similarly, at least one of the above-described positive electrode active materials and other positive electrode active material may be used as a mixture. Examples of the other positive electrode active material include transition metal oxides that are not mentioned above, transition metal phosphate compounds, transition metal silicate compounds, and transition metal borate compounds.

Thereamong, lithium-manganese composite oxides having a spinel structure and lithium-containing transition metal phosphate compounds having an olivine structure are preferred. Specific examples of the lithium-manganese composite oxides having a spinel structure include $LiMn_2O_4$, $LiM_{n1}.8Al_{0.2}O_4$, and $LiMn_{1.5}Ni_{0.5}O_4$. These lithium-manganese composite oxides have the most stable structure and are thus unlikely to release oxygen even in the event of malfunction of the nonaqueous electrolyte battery, providing excellent safety.

The transition metals of the lithium-containing transition metal phosphate compounds are preferably V, Ti, Cr, Mn, Fe, Co, Ni, Cu or the like, and specific examples of such compounds include: iron phosphates, such as $LiFePO_4$, $Li_3Fe_2(PO_4)_3$, and $LiFeP_2O_7$; cobalt phosphates, such as $LiCoPO_4$; manganese phosphates, such as $LiMnPO_4$; and these lithium transition metal phosphate compounds in which some of the transition metal atoms contained as a main constituent are substituted with other metal such as Al, Ti, V, Cr, Mn, Fe, Co, Li, Ni, Cu, Zn, Mg, Ga, Zr, Si, Nb, Mo, Sn, or W.

Thereamong, a lithium iron phosphate compound is preferred since iron is not only abundant in terms of resource amount and thus an extremely cheap metal but also hardly hazardous. In other words, among the above-described specific examples, $LiFePO_4$ can be mentioned as a more preferred specific example.

(2) Surface Coating

The above-described positive electrode substance may be used in the form that a substance having a composition different from that of the substance mainly constituting the positive electrode active material is adhered to the surface (hereinafter, such a substance is referred to as "surface adhering substance" as appropriate). Examples of the surface adhering substance include: oxides, such as aluminum oxide, silicon oxide, titanium oxide, zirconium oxide, magnesium oxide, calcium oxide, boron oxide, antimony oxide, and bismuth oxide; sulfates, such as lithium sulfate, sodium sulfate, potassium sulfate, magnesium sulfate, calcium sulfate, and aluminum sulfate; carbonates, such as lithium carbonate, calcium carbonate, and magnesium carbonate; and carbon.

The surface adhering substance can be adhered to the surface of the positive electrode active material by, for example, a method in which the surface adhering substance is dissolved or suspended in a solvent, and the resulting solution or suspension is added to and impregnated into the positive electrode active material, followed by drying; a

method in which a precursor of the surface adhering substance is dissolved or suspended in a solvent, and the resulting solution or suspension is added to and impregnated into the positive electrode active material and then allowed to react by heating or the like; or a method in which a precursor of the positive electrode active material is fired simultaneously with addition of the surface adhering substance thereto. For adhesion of carbon, a method of mechanically adhering a carbonaceous material afterward in the form of activated carbon or the like may be employed.

The mass of the surface adhering substance on the surface of the positive electrode active material is preferably 0.1 ppm or more, more preferably 1 ppm or more, still more preferably 10 ppm or more, but preferably 20% or less, more preferably 10% or less, still more preferably 5% or less, with respect to the mass of the positive electrode active material.

By the presence of the surface adhering substance, an oxidation reaction of the nonaqueous electrolyte solution on the surface of the positive electrode active material can be suppressed, so that the battery life can be extended. Further, when the amount of adhering substance is in the above-described range, the effect thereof can be sufficiently expressed, and this makes the resistance unlikely to increase, without inhibiting the movement of lithium ions in and out of the positive electrode active material.

(3) Shape

The particles of the positive electrode active material particles may have any conventionally used shape, such as a lump shape, a polyhedral shape, a spherical shape, an ellipsoidal shape, a plate shape, a needle shape, or a columnar shape. Further, primary particles may be aggregated to form secondary particles that have a spherical or ellipsoidal shape.

(4) Method of Producing Positive Electrode Active Substance

A method of producing the positive electrode active material is not particularly restricted within a range that does not depart from the gist of the present invention. Examples thereof include several methods, and a general method of producing an inorganic compound may be employed.

Particularly, for the production of a spherical or ellipsoidal active material, a variety of methods can be considered. One example thereof is a method in which a transition metal raw material substance (e.g., a nitrate salt or sulfate salt of a transition metal) and, as required, a raw material substance of other element are dissolved, or pulverized and dispersed in a solvent such as water, the pH of the resulting solution or dispersion is adjusted with stirring to produce and recover a spherical precursor, and this precursor is subsequently dried as required, after which a Li source such as LiOH, $Li_2CO_3$ or $LiNO_3$ is added thereto and the resultant is fired at a high temperature to obtain an active material.

Another example is a method in which a transition metal raw material substance (e.g., a nitrate salt, sulfate salt, hydroxide, oxide or the like of a transition metal) and, as required, a raw material substance of other element are dissolved, or pulverized and dispersed in a solvent such as water, and the resulting solution or dispersion is dried and shaped using a spray dryer or the like to produce a spherical or ellipsoidal precursor, after which a Li source such as LiOH, $Li_2CO_3$ or $LiNO_3$ is added thereto and the resultant is fired at a high temperature to obtain an active material.

Yet another example is a method in which a transition metal raw material substance (e.g., a nitrate salt, sulfate salt, hydroxide, oxide or the like of a transition metal), a Li source such as LiOH, $Li_2CO_3$ or $LiNO_3$ and, as required, a raw material substance of other element are dissolved, or pulverized and dispersed in a solvent such as water, and the resulting solution or dispersion is dried and shaped using a spray dryer or the like to produce a spherical or ellipsoidal precursor, after which this precursor is fired at a high temperature to obtain an active material.

<2-4-2. Constitution and Production Method of Positive Electrode>

The constitution of the positive electrode used in the present invention and a method of producing the positive electrode will now be described.

(Method of Producing Positive Electrode)

The positive electrode is produced by forming a positive electrode active material layer containing particles of the positive electrode active material and a binder on a current collector. Such production of the positive electrode using the positive electrode active material can be carried out by any known method. For example, a positive electrode active material layer is formed on a current collector by dry-mixing the positive electrode active material and a binder with, as required, a conductive material, a thickening agent and the like to form a sheet and subsequently press-bonding this sheet onto a positive electrode current collector, or by dissolving or dispersing these materials in a liquid medium to prepare a slurry and subsequently applying and drying this slurry onto a positive electrode current collector, whereby the positive electrode can be obtained.

The content of the positive electrode active material in the positive electrode active material layer is preferably 60% by mass or higher, more preferably 70% by mass or higher, still more preferably 80% by mass or higher, but preferably 99.9% by mass or less, more preferably 99% by mass or less. When the content of the positive electrode active material is in this range, a sufficient capacitance can be ensured. In addition, the resulting positive electrode has a sufficient strength. In the present invention, a single kind of positive electrode active material powder may be used alone, or two or more kinds thereof having different compositions or physical properties may be used in any combination at any ratio. When two or more kinds of active materials are used in combination, it is preferred to use the above-described composite oxide containing lithium and manganese as a powder component. In large-sized batteries for automobile applications and the like where a large capacity is required and a large amount of active material is used, cobalt and nickel are not preferred from the cost standpoint since they are not abundant in terms of resource amount and are thus expensive metals; therefore, it is desirable to use manganese, which is a less expensive transition metal, as a main component.

(Conductive Material)

As the conductive material, any known conductive material can be used. Specific examples thereof include metal materials, such as copper and nickel; and carbonaceous materials, such as graphites (e.g., natural graphites and artificial graphites), carbon blacks (e.g., acetylene black), and amorphous carbon (e.g., needle coke). Any of these conductive materials may be used singly, or two or more thereof may be used in any combination at any ratio.

The content of the conductive material in the positive electrode active material layer is preferably 0.01% by mass or higher, more preferably 0.1% by mass or higher, still more preferably 1% by mass or higher, but preferably 50% by mass or less, more preferably 30% by mass or less, still more preferably 15% by mass or less. When the content of the conductive material is in this range, a sufficient electrical conductivity can be ensured. In addition, a reduction in the battery capacity is likely to be inhibited.

(Binder)

The binder used in the production of the positive electrode active material layer is not particularly restricted as long as it is a material that is stable against the nonaqueous electrolyte solution and the solvent used in the electrode production.

When a coating method is employed, the binder is not particular restricted as long as it is a material that can be dissolved or dispersed in the liquid medium used in the electrode production, and specific examples of such a binder include: resin-based polymers, such as polyethylene, polypropylene, polyethylene terephthalate, polymethyl methacrylate, aromatic polyamides, cellulose, and nitrocellulose; rubbery polymers, such as SBR (styrene-butadiene rubbers), NBR (acrylonitrile-butadiene rubbers), fluororubbers, isoprene rubbers, butadiene rubbers, and ethylene-propylene rubbers; thermoplastic elastomeric polymers, such as styrene-butadiene-styrene block copolymers and hydrogenation products thereof, EPDM (ethylene-propylene-diene terpolymers), styrene-ethylene-butadiene-ethylene copolymers, styrene-isoprene-styrene block copolymers, and hydrogenation products thereof; soft resinous polymers, such as syndiotactic 1,2-polybutadiene, polyvinyl acetate, ethylene-vinyl acetate copolymers, and propylene-$\alpha$-olefin copolymers; fluorine-based polymers, such as polyvinylidene fluoride (PVdF), polytetrafluoroethylene, and polytetrafluoroethylene-ethylene copolymers; and polymer compositions having ionic conductivity for alkali metal ions (particularly lithium ions). Any of these substances may be used singly, or two or more thereof may be used in any combination at any ratio.

The content of the binder in the positive electrode active material layer is preferably 0.1% by mass or higher, more preferably 1% by mass or higher, still more preferably 3% by mass or higher, but preferably 80% by mass or less, more preferably 60% by mass or less, still more preferably 40% by mass or less, particularly preferably 10% by mass or less. When the ratio of the binder is in this range, the positive electrode active material can be sufficiently retained, and the mechanical strength of the positive electrode can be ensured; therefore, favorable battery performance such as cycle characteristics are attained. This also leads to avoidance of a reduction in the battery capacity and conductivity.

(Liquid Medium)

The type of the liquid medium used in the preparation of a slurry for forming the positive electrode active material layer is not particularly restricted as long as it is a solvent that is capable of dissolving or dispersing the positive electrode active material, the conductive material and the binder as well as a thickening agent used as required, and either an aqueous solvent or an organic solvent may be used.

Examples of the aqueous solvent include water, and mixed media of alcohol and water. Examples of the organic solvent include: aliphatic hydrocarbons, such as hexane; aromatic hydrocarbons, such as benzene, toluene, xylene, and methylnaphthalene; heterocyclic compounds, such as quinoline and pyridine; ketones, such as acetone, methyl

ethyl ketone, and cyclohexanone; esters, such as methyl acetate and methyl acrylate; amines, such as diethylenetriamine and *N*,*N*-dimethylaminopropylamine; ethers, such as diethyl ether and tetrahydrofuran (THF); amides, such as *N*-methylpyrrolidone (NMP), dimethylformamide, and dimethylacetamide; and aprotic polar solvents, such as hexamethylphosphoramide and dimethyl sulfoxide. Any of these media may be used singly, or two or more thereof may be used in any combination at any ratio.

(Thickening Agent)

When an aqueous medium is used as the liquid medium for the formation of a slurry, it is preferred to prepare a slurry using a thickening agent and a latex such as a styrene-butadiene rubber (SBR). The thickening agent is usually used for the purpose of adjusting the viscosity of the resulting slurry.

The thickening agent is not particularly restricted as long as it does not markedly limit the effects of the present invention, and specific examples of the thickening agent include carboxymethyl cellulose, methyl cellulose, hydroxymethyl cellulose, ethyl cellulose, polyvinyl alcohol, oxidized starch, phosphorylated starch, casein, and salts thereof. Any of these thickening agents may be used singly, or two or more thereof may be used in any combination at any ratio.

In cases where a thickening agent is used, the ratio thereof with respect to the positive electrode active material is preferably 0.1% by mass or higher, more preferably 0.5% by mass or higher, still more preferably 0.6% by mass or higher, but preferably 5% by mass or lower, more preferably 3% by mass or lower, still more preferably 2% by mass or lower. When the ratio of the thickening agent is in this range, a favorable coating property is attained, and the resulting positive electrode active material layer has a sufficient ratio of the active material; therefore, problems of a reduction in the battery capacity and an increase in the resistance between the positive electrode active material are likely to be avoided.

(Consolidation)

The positive electrode active material layer obtained by applying and drying the above-described slurry onto a current collector is preferably consolidated by means of hand pressing, roller pressing or the like so as to increase the packing density of the positive electrode active material. The density of the positive electrode active material layer is preferably 1 $g \cdot cm^{-3}$ or higher, more preferably 1.5 $g \cdot cm^{-3}$ or higher, particularly preferably 2 $g \cdot cm^{-3}$ or higher, but preferably 4 $g \cdot cm^{-3}$ or lower, more preferably 3.5 $g \cdot cm^{-3}$ or lower, particularly preferably 3 $g \cdot cm^{-3}$ or lower.

When the density of the positive electrode active material layer is in this range, the permeability of the nonaqueous electrolyte solution to the vicinity of the interface between the current collector and the active material is not reduced, so that favorable charge-discharge characteristics are attained particularly at high current densities. In addition, neither a reduction in the conductivity between the active materials nor an increase in the battery resistance is likely to occur.

(Current Collector)

The material of the positive electrode current collector is not particularly restricted, and any known material can be used. Specific examples thereof include: metal materials, such as aluminum, stainless steel, nickel-plated steel, titanium, and tantalum; and carbonaceous materials, such as carbon cloth and carbon paper. Thereamong, a metal material, particularly aluminum, is preferred.

When the current collector is a metal material, the current collector may have any shape of, for example, a metal foil, a metal cylinder, a metal coil, a metal sheet, a metal thin film, an expanded metal, a punched metal, and a foamed metal and, when the current collector is a carbonaceous material, examples thereof include a carbon sheet, a carbon thin film, and a carbon cylinder. Thereamong, the current collector is preferably a metal thin film. As appropriate, the current collector may be in the form of a mesh.

The current collector may have any thickness; however, the thickness is preferably 1 $\mu m$ or greater, more preferably 3 $\mu m$ or greater, still more preferably 5 $\mu m$ or greater, but preferably 1 mm or less, more preferably 100 $\mu m$ or less, still more preferably 50 $\mu m$ or less. When the thickness of the current collector is in this range, a sufficient strength required as a current collector can be ensured. In addition, the current collector has good ease of handling.

The thickness ratio of the current collector and the positive electrode active material layer is not particularly restricted; however, the value of "(Thickness of active material layer on one side immediately before injection of nonaqueous electrolyte solution)/(Thickness of current collector)" is preferably 150 or smaller, more preferably 20 or smaller, particularly preferably 10 or smaller, but preferably 0.1 or larger, more preferably 0.4 or larger, particularly preferably 1 or larger.

When the thickness ratio of the current collector and the positive electrode active material layer is in this range, heat generation by the current collector due to Joule's heat during high-current-density charging/discharging is unlikely

to occur. In addition, the volume ratio of the current collector with respect to the positive electrode active material is hardly increased, so that a reduction in the battery capacity can be inhibited.

(Electrode Area)

From the standpoint of improving the stability under high-output and high-temperature conditions, the positive electrode active material layer preferably has a large area relative to the outer surface area of a battery outer casing. Specifically, the total area of the positive electrode is, in terms of area ratio, preferably 20 times or larger, more preferably 40 times or larger, with respect to the surface area of the outer casing of the nonaqueous electrolyte battery. The "outer surface area of outer casing" refers to, in the case of a closed-bottom prism-shaped casing, a total area calculated from the length, the width and the thickness of a portion of the casing that is filled with a power-generating element, excluding the projecting parts of the terminals. In the case of a closed-bottom cylindrical casing, the "outer surface area of outer casing" refers to a geometric surface area determined by approximation of a portion of the casing that is filled with a power-generating element, excluding the projecting parts of the terminals, to a cylinder. The "the total area of the positive electrode", which is a geometric surface area of a positive electrode mixture layer facing a mixture layer containing the negative electrode active material, refers to a sum of the areas that are separately calculated for each side in a structure in which positive electrode layer mixture layers are formed on both sides via a current collector foil.

(Discharge Capacity)

When the nonaqueous electrolyte solution of the present invention is used, the capacitance of the elements of the nonaqueous electrolyte battery that are housed in a single battery casing (the capacitance measured in the course of discharging the battery from a fully-charged state to a discharged state) is preferably 1 ampere hour (Ah) or higher since this leads to an enhanced effect of improving the low-temperature discharge characteristics. Accordingly, the positive electrode plate is designed to have a discharge capacity of preferably 3 Ah (ampere hour) or higher, more preferably 4 Ah or higher, but preferably 100 Ah or less, more preferably 70 Ah or less, particularly preferably 50 Ah or less, in a fully-charged state.

When the discharge capacity is in this range, a voltage drop caused by electrode reaction resistance during extraction of a large current is not overly large, so that a reduction in the power efficiency can be inhibited. In addition, since the temperature distribution caused by internal heat generation of the battery during pulse charging and discharging is not excessively wide, phenomena of deterioration in the durability against repeated charging and discharging and a reduction in the heat dissipation efficiency against abrupt heat generation in the event of a defect such as overcharging or internal short-circuiting can be avoided.

(Thickness of Positive Electrode Plate)

The thickness of the positive electrode plate is not particularly restricted; however, from the standpoint of attaining a high capacity and a high output as well as excellent rate characteristics, the thickness of the positive electrode active material layer excluding the thickness of the current collector is preferably 10 μm or greater, more preferably 20 μm or greater, but preferably 200 μm or less, more preferably 100 μm or less, on one side of the current collector.

<2-5. Separator>

A separator is usually arranged between the positive electrode and the negative electrode for the purpose of inhibiting a short circuit. In this case, the separator is usually impregnated with the nonaqueous electrolyte solution of the present invention.

The material and the shape of the separator are not particularly restricted as long as the separator does not markedly impair the effects of the present invention, and any known material and shape can be employed. Particularly, a separator formed from a material stable against the nonaqueous electrolyte solution of the present invention, such as a resin, a glass fiber or an inorganic material, can be used, and it is preferred to use a separator in the form of, for example, a porous sheet or nonwoven fabric that has excellent liquid retainability.

As the material of a resin or glass-fiber separator, for example, polyolefins such as polyethylene and polypropylene, polytetrafluoroethylenes, polyether sulfones, and glass filters can be used. Thereamong, glass filters and polyolefins are preferred, and polyolefins are more preferred. Any of these materials may be used singly, or two or more thereof may be used in any combination at any ratio.

The separator may have any thickness; however, the thickness is usually 1 μm or greater, preferably 5 μm or greater, more preferably 10 μm or greater, but usually 50 μm or less, preferably 40 μm or less, more preferably 30 μm or less. When the separator is thinner than this range, the insulation and the mechanical strength may be reduced.

Meanwhile, when the separator is thicker than this range, not only the battery performance such as the rate characteristics may be deteriorated, but also the energy density of the nonaqueous electrolyte battery as a whole may be reduced.

In cases where a porous material such as a porous sheet or a nonwoven fabric is used as the separator, the porosity of the separator may be set arbitrarily; however, it is usually 20% or higher, preferably 35% or higher, more preferably 45% or higher, but usually 90% or lower, preferably 85% or lower, more preferably 75% or lower. When the porosity is lower than this range, the membrane resistance is increased, and the rate characteristics thus tend to be deteriorated. Meanwhile, when the porosity is higher than this range, the mechanical strength and the insulation of the separator tend to be reduced.

The average pore size of the separator may also be set arbitrarily; however, it is usually 0.5 μm or smaller, preferably 0.2 μm or smaller, but usually 0.05 μm or larger. When the average pore size is larger than this range, a short circuit is likely to occur. Further, when the average pore size is smaller than this range, the membrane resistance is increased, and this may lead to deterioration of the rate characteristics.

Meanwhile, as the material of an inorganic separator, for example, an oxide such as alumina or silicon dioxide, a nitride such as aluminum nitride or silicon nitride, or a sulfate such as barium sulfate or calcium sulfate can be used, and the inorganic separator may have a particulate shape or a fibrous shape.

With regard to the form of the separator, a nonwoven fabric, a woven fabric, or a thin film such as a microporous film may be used. As a thin-film separator, one having a pore size of 0.01 to 1 μm and a thickness of 5 to 50 μm is preferably used. Aside from such an independent thin-film separator, a separator that is formed as, with the use of a resin binder, a composite porous layer containing particles of the above-described inorganic material on the surface layer of the positive electrode and/or the negative electrode, can be used. For example, on both sides of the positive electrode, a porous layer may be formed using alumina particles having a 90% particle size of smaller than 1 μm along with a fluorine resin as a binder.

<2-6. Battery Design>

[Electrode Group]

An electrode group may have either a layered structure in which the above-described positive electrode plate and negative electrode plate are layered with the above-described separator being interposed therebetween, or a wound structure in which the above-described positive electrode plate and negative electrode plate are spirally wound with the above-described separator being interposed therebetween. The volume ratio of the electrode group with respect to the internal volume of the battery (this volume ratio is hereinafter referred to as "electrode group occupancy") is usually 40% or higher, preferably 50% or higher, but usually 90% or lower, preferably 80% or lower. From the standpoint of the battery capacity, the lower limit of the electrode group occupancy is preferably controlled in this range. Further, from the standpoint of the various properties of the battery, such as the repeated charge-discharge performance and the high-temperature storage characteristics, as well as from the standpoint of avoiding activation of a gas release valve that relieves the internal pressure to the outside, the upper limit of the electrode group occupancy is preferably controlled in this range so as to ensure a sufficient gap space. When the amount of gap space is excessively small, there are cases where a rise8 in the battery temperature causes swelling of members and increases the vapor pressure of the electrolyte liquid component, as a result of which the internal pressure is increased to deteriorate the various properties of the battery, such as the repeated charge-discharge performance and the high-temperature storage characteristics, and to activate a gas release valve for relieving the internal pressure to the outside.

(Current Collector Structure)

The current collector structure is not particularly restricted; however, in order to more effectively realize an improvement in the discharge characteristics attributed to the nonaqueous electrolyte solution of the present invention, it is preferred to adopt a structure that reduces the resistance of wiring and joint parts. By reducing the internal resistance in this manner, the effects of using the nonaqueous electrolyte solution of the present invention are particularly favorably exerted.

In an electrode group having the above-described layered structure, the metal core portions of the respective electrode layers are preferably bundled and welded to a terminal. When the area of a single electrode is large, the internal resistance is high; therefore, it is also preferred to reduce the resistance by arranging plural terminals in each electrode. In an electrode group having the above-described wound structure, the internal resistance can be reduced by arranging plural lead structures on each of the positive electrode and the negative electrode and bundling them to a terminal.

[Protective Element]

Examples of a protective element include a PTC (Positive Temperature Coefficient) element whose resistance increases in the event of abnormal heat generation or excessive current flow, a thermal fuse, a thermistor, and a valve (current cutoff valve) that blocks a current flowing into a circuit in response to a rapid increase in the internal pressure or internal temperature of the battery in the event of abnormal heat generation. The protective element is preferably selected from those that are not activated during normal use at a high current and, from the standpoint of attaining a high output, it is more preferred to design the battery such that neither abnormal heat generation nor thermal runaway occurs even without a protective element.

[Outer Package]

The nonaqueous electrolyte battery of the present invention is usually constructed by housing the above-described nonaqueous electrolyte solution, negative electrode, positive electrode, separator and the like in an outer package (outer casing). This outer package is not restricted, and any known outer package can be employed as long as it does not markedly impair the effects of the present invention.

The material of the outer casing is not particularly restricted as long as it is a substance that is stable against the nonaqueous electrolyte solution to be used. Specifically, a metal such as a nickel-plated steel sheet, stainless steel, aluminum, an aluminum alloy, a magnesium alloy, nickel or titanium, or a laminated film composed of a resin and an aluminum foil can be preferably used.

Examples of an outer casing using any of the above-described metals include those having a hermetically sealed structure obtained by welding metal pieces together by laser welding, resistance welding or ultrasonic welding, and those having a caulked structure obtained using the above-described metals via a resin gasket. Examples of an outer casing using the above-described laminated film include those having a hermetically sealed structure obtained by heat-fusing resin layers together. In order to improve the sealing performance, a resin different from the resin used in the laminated film may be interposed between the resin layers. Particularly, in the case of forming a sealed structure by heat-fusing resin layers via a collector terminal, since it involves bonding between a metal and a resin, a polar group-containing resin or a resin modified by introduction of a polar group is preferably used as the resin to be interposed.

The shape of the outer package may also be selected arbitrarily, and the outer package may have any of, for example, a cylindrical shape, a prismatic shape, a laminated shape, a coin shape, and a large-sized shape.

EXAMPLES

The present invention will now be described more concretely by way of Examples and Comparative Examples; however, the present invention is not restricted thereto within the gist of the present invention.

Compounds 1 to 6 used in Examples and Comparative Examples are shown below. The compound 1 corresponds to the cyclic carbonate having an unsaturated carbon-carbon bond that is defined in the present specification; the compound 2 corresponds to the compound represented by Formula (A) that is defined in the present specification; the compound 3 and the compound 6 each correspond to the compound represented by Formula (B) that is defined in the present specification; the compound 4 corresponds to the compound represented by Formula (C) that is defined in the present specification; and the compound 5 corresponds to the fluorinated salt defined in the present specification.

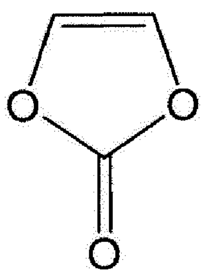

(Compound 1)

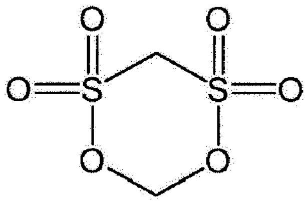

(Compound 2)

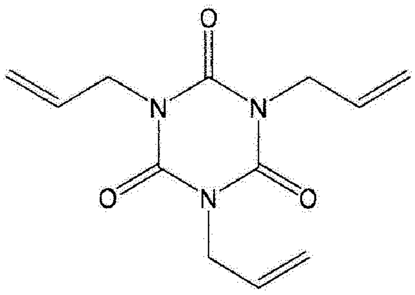

(Compound 3)

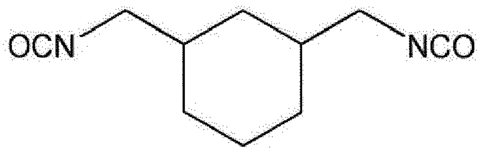

(Compound 4)

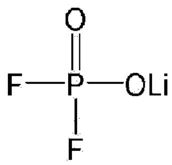

(Compound 5)

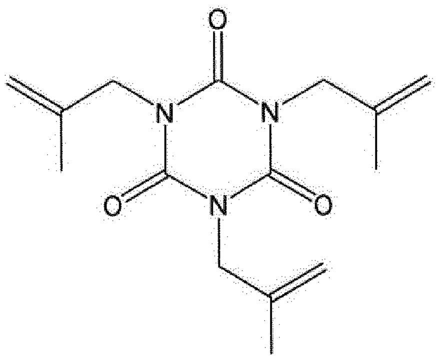

(Compound 6)

<Examples 1-1 to 1-12 and Comparative Examples 1-1 to 1-9>

[Production of Positive Electrode]

A slurry was prepared by mixing 90 parts by mass of lithium-nickel-cobalt-manganese composite oxide ($Li_{1.0}Ni_{0.5}CO_{0.2}Mn_{0.3}O_2$) as a positive electrode active material, 7 parts by mass of acetylene black as a conductive material and 3 parts by mass of polyvinylidene fluoride (PVdF) as a binder in an *N*-methylpyrrolidone solvent using a disperser. Both sides of a 15 μm-thick aluminum foil were uniformly coated with this slurry, and this aluminum foil was subsequently dried and then pressed to obtain a positive electrode.

[Production of Negative Electrode]

To 98 parts by mass of natural graphite, 1 part by mass of an aqueous dispersion of sodium carboxymethyl

cellulose (concentration of sodium carboxymethyl cellulose = 1% by mass) and 1 part by mass of an aqueous dispersion of styrene-butadiene rubber (concentration of styrene-butadiene rubber = 50% by mass) were added as a thickening agent and a binder, respectively, and these materials were mixed using a disperser to prepare a slurry. The thus obtained slurry was coated and dried onto one side of a 10 μm-thick copper foil, and this copper foil was subsequently pressed to obtain a negative electrode.

[Preparation of Nonaqueous Electrolyte Solutions]

Under a dry argon atmosphere, thoroughly dried $LiPF_6$ was dissolved as an electrolyte at 1.15 mol/L (in terms of the concentration in the resulting nonaqueous electrolyte solution) in a mixture of ethylene carbonate (EC), diethyl carbonate (DEC) and ethyl methyl carbonate (EMC) (volume ratio EC:DEC:EMC = 3:6:1), and 1.15% by mass (in terms of the concentration in the resulting nonaqueous electrolyte solution) of fluoroethylene carbonate (FEC) was further added thereto (the resultant is hereinafter referred to as "standard electrolyte solution 1"). To the thus obtained standard electrolyte solution 1, the compounds 1 to 6 were added as additives in the respective amounts shown in Table 1 below to prepare nonaqueous electrolyte solutions. It is noted here that, in Table 1, "Content (% by mass)" indicates an amount contained in a total of 100% by mass of each nonaqueous electrolyte solution.

[Production of Nonaqueous Electrolyte Batteries]

A battery element was prepared by laminating the above-obtained positive electrode and negative electrode along with a polyethylene separator in the order of the negative electrode, the separator, and the positive electrode. This battery element was inserted into a pouch made of a laminated film obtained by coating both sides of an aluminum sheet (thickness: 40 μm) with a resin layer, with the terminals of the positive and negative electrodes protruding out of the pouch. Thereafter, each of the above-prepared nonaqueous electrolyte solutions was injected into the pouch, and the pouch was subsequently vacuum-sealed, whereby a laminate-type nonaqueous electrolyte battery was produced.

<Evaluation of Nonaqueous Electrolyte Batteries>

[Initial Conditioning]

In a 25°C thermostat chamber, each nonaqueous electrolyte battery produced by the above-described method was charged to 3.2V at a constant current equivalent to 0.1 C (a current value of 1 C means the current value at which charging or discharging of the battery requires one hour; the same applies below), and subsequently subjected to constant current-constant voltage charging (hereinafter, referred to as "CC-CV charging") up to 4.2 V at 0.2 C. The nonaqueous electrolyte battery was then maintained at 45°C for 120 hours to perform aging, after which the nonaqueous electrolyte battery was discharged to 2.5 V at 0.2 C and stabilized. Thereafter, the nonaqueous electrolyte battery was CC-CV charged to 4.2 V at 0.2 C and then discharged to 2.5 V at 0.2 C, whereby initial conditioning was completed.

After the initial conditioning, the battery was CC-CV charged at 0.2 C such that the battery had a half of the initial discharge capacity. This battery was discharged at each current value of 1.0 C, 2.0 C and 3.0 C at 25°C, and the voltage was measured at a point of 5 seconds into each discharging process. An average value of the slopes of the thus obtained current-voltage straight lines at 1.0 C, 2.0 C and 3.0 C was defined as the initial resistance of the battery.

<Evaluation of Nonaqueous Electrolyte Batteries>

[Charged Storage Test]

After the initial conditioning, each laminate-type nonaqueous electrolyte battery was again CC-CV charged to 4.2 V at 0.2C and subsequently stored at a high temperature of 60°C for 168 hours. The battery was thoroughly cooled and then immersed in an ethanol bath to measure the volume, and the amount of generated gas, which was determined from the volume change before and after the storage test, was defined as "amount of gas generated during charged storage".

Further, after the storage test, the nonaqueous electrolyte battery was discharged to 2.5 V at 0.2 C and subsequently CC-CV charged at 0.2 C such that the battery had a half of the initial discharge capacity, and the initial resistance of the battery after the storage test was determined. The "initial resistance increase rate" was calculated using the following formula (4).

$$\text{Internal resistance increase rate} = [(\text{Internal resistance after storage test})/(\text{Internal resistance after initial conditioning})] \times 100\% \quad (4)$$

Table 1 below shows the ratio of the amount of gas generated during charged storage and the ratio of the internal resistance increase rate, taking the amount of gas generated during charged storage and the internal resistance increase rate in Comparative Example 1-1 as 100, respectively.

Table 1

| | Content of Compound 1 (% by mass) | Content of Compound 2 (% by mass) | Content of Compound 3 (% by mass) | Content of Compound 4 (% by mass) | Content of Compound 5 (% by mass) | Content of Compound 6 (% by mass) | Ratio of amount of gas generated during charged storage | Ratio of internal resistance increase rate |
|---|---|---|---|---|---|---|---|---|
| Example 1-1 | 1.25 | 1.00 | 0.15 | - | - | - | 67 | 98 |
| Examplel-2 | 1.25 | 1.00 | 0.20 | - | - | - | 68 | 99 |
| Examplel-3 | 1.25 | 1.00 | 0.30 | - | - | - | 61 | 99 |
| Examplel-4 | 1.00 | 1.00 | 0.20 | - | - | - | 64 | 99 |
| Example1-5 | 1.25 | 1.00 | - | 0.20 | - | - | 72 | 98 |
| Example1-6 | 0.75 | 1.00 | 0.20 | 0.20 | - | - | 46 | 96 |
| Examplel-7 | 1.25 | 1.00 | - | 0.30 | - | - | 61 | 98 |
| Example1-8 | 1.25 | 1.00 | - | - | - | 0.23 | 79 | 98 |
| Example1-9 | 0.75 | 0.50 | - | 0.30 | 0.50 | - | 33 | 99 |
| Example 1-10 | 1.25 | 1.00 | 0.40 | - | - | - | 58 | 97 |
| Example 1-11 | 1.25 | 1.00 | - | - | - | 0.46 | 63 | 97 |
| Example I-12 | 0.75 | 1.00 | 0.15 | 0.15 | - | - | 49 | 97 |
| Comparative Example 1-1 | 1.65 | 1.00 | - | - | - | - | 100 | 100 |
| Comparative Example 1-2 | 1.65 | 1.00 | 0.20 | - | - | - | 91 | 102 |
| Comparative Example 1-3 | 1.65 | 1.00 | 0.30 | - | - | - | 85 | 103 |
| Comparative Example 1-4 | 1.25 | 1.00 | 0.50 | - | - | - | 66 | 103 |
| Comparative Example1-5 | 1.25 | 1.00 | - | 0.50 | - | - | 64 | 100 |
| Comparative Example1-6 | 1.25 | - | 0.20 | - | - | - | 72 | 113 |

(continued)

| | Content of Compound 1 (% by mass) | Content of Compound 2 (% by mass) | Content of Compound 3 (% by mass) | Content of Compound 4 (% by mass) | Content of Compound 5 (% by mass) | Content of Compound 6 (% by mass) | Ratio of amount of gas generated during charged storage | Ratio of internal resistance increase rate |
|---|---|---|---|---|---|---|---|---|
| Comparative Example 1-7 | - | 1.00 | 0.20 | - | - | - | 54 | 102 |
| Comparative Example1-8 | 1.25 | 1.00 | - | - | - | - | 95 | 100 |
| Comparative Example1-9 | 1.25 | 1.00 | - | 0.75 | - | - | 69 | 102 |

As compared to Comparative Examples 1-1 and 1-8 where only the compounds 1 and 2 were added, not only the amount of gas generated during charged storage was largely reduced but also the internal resistance was lowered in all of Examples 1-1 to 1-5, 1-7 and 1-10 where the compound 3 or 4 was further added in an amount of 0.49% by mass or less. Particularly, in Examples 1-6 and 1-12 where the compounds 3 and 4 were both incorporated, the battery characteristics were further improved as compared to those cases where only one of them was incorporated. Moreover, also in Examples 1-8 and 1-11 where the compound 6 was added in an amount of 0.49% by mass or less in place of the compound 3, the battery characteristics were improved in the same manner as in those cases of using the compound 3.

On the other hand, in Comparative Examples 1-2 and 1-3 where all of the compounds 1 to 3 were incorporated but the content of the compound 1 was higher than 1.5% by mass, although the amount of gas generated during charged storage was reduced as compared to Comparative Examples 1-1 and 1-8, the extent of the improvement was not as large as that of Examples 1-1 to 1-4, and the internal resistance was rather increased.

In addition, in Comparative Examples 1-4, 1-5 and 1-9 where all of the compounds 1 to 3 were incorporated but the content of the compound 3 or 4 was higher than 0.49% by mass, although the amount of gas generated during charged storage was improved to a similar extent as in Examples 1-1 to 1-4 and 1-7, the internal resistance was the same or rather increased as compared to Comparative Examples 1-1 and 1-8.

Moreover, in Comparative Examples 1-6 and 1-7 where the compound 3 was added in an amount of 0.49% by mass or less but only one of the compounds 1 and 2 was incorporated, although the amount of gas generated during charged storage was improved to a similar extent as in Examples 1-1 to 1-4, the internal resistance was rather increased as compared to Comparative Examples 1-1 and 1-8.

<Examples 2-1 to 2-3 and Comparative Example 2-1>

[Production of Positive Electrode]

A slurry was prepared by mixing 94 parts by mass of lithium-nickel-cobalt-manganese composite oxide ($Li_{1.0}Ni_{0.6}Co_{0.2}Mn_{0.2}O_2$) as a positive electrode active material, 3 parts by mass of acetylene black as a conductive material and 3 parts by mass of polyvinylidene fluoride (PVdF) as a binder in an *N*-methylpyrrolidone solvent using a disperser. Both sides of a 15 μm-thick aluminum foil were uniformly coated with this slurry, and this aluminum foil was subsequently dried and then pressed to obtain a positive electrode.

[Production of Negative Electrode]

A negative electrode was produced and used in the same manner as in Example 1-1.

[Preparation of Nonaqueous Electrolyte Solutions]

To the standard electrolyte solution 1, the compounds 1 to 4 were added as additives in the respective amounts shown in Table 2 below to prepare nonaqueous electrolyte solutions. It is noted here that, in Table 2, "Content (% by mass)" indicates an amount contained in a total of 100% by mass of each nonaqueous electrolyte solution.

[Production of Nonaqueous Electrolyte Batteries]

A battery element was prepared by laminating the above-obtained positive electrode and negative electrode along with a polyethylene separator in the order of the negative electrode, the separator, and the positive electrode. This battery element was inserted into a pouch made of a laminated film obtained by coating both sides of an aluminum sheet (thickness: 40 μm) with a resin layer, with the terminals of the positive and negative electrodes protruding out of the pouch. Thereafter, each of the above-prepared nonaqueous electrolyte solutions was injected into the pouch, and the pouch was subsequently vacuum-sealed, whereby a laminate-type nonaqueous electrolyte battery was produced.

<Evaluation of Nonaqueous Electrolyte Batteries>

For each of the thus produced nonaqueous electrolyte batteries, the amount of gas generated during charged storage and the internal resistance increase rate were determined in the same manner as in Example 1-1. Table 2 below shows the ratio of the amount of gas generated during charged storage and the ratio of the internal resistance increase rate, taking the amount of gas generated during charged storage and the internal resistance increase rate in Comparative Example 2-1 as 100, respectively.

Table 2

| | Content of Compound 1 (% by mass) | Content of Compound 2 (% by mass) | Content of Compound 3 (% by mass) | Content of Compound 4 (% by mass) | Ratio of amount of gas generated during charged storage | Ratio of internal resistance increase rate |
|---|---|---|---|---|---|---|
| Example2-1 | 1.25 | 1.00 | 0.20 | - | 54 | 97 |
| Example2-2 | 1.25 | 1.00 | - | 0.20 | 51 | 95 |
| Example2-3 | 0.75 | 1.00 | 0.15 | 0.15 | 42 | 94 |
| Comparative Example2-1 | 1.65 | 1.00 | - | - | 100 | 100 |

From the results shown in Table 2, it is apparent that the effects attributed to the use of the nonaqueous electrolyte solution of the present invention can be obtained even when the Ni content in the positive electrode active material is higher than in Example 1.

<Examples 3-1 to 3-3 and Comparative Example 3-1>

[Production of Positive Electrode]

A slurry was prepared by mixing 85% by mass of lithium-nickel-manganese-cobalt composite oxide ($Li_{1.0}Ni_{0.33}Co_{0.33}Mn_{0.33}O_2$) as a positive electrode active material, 10% by mass of acetylene black as a conductive material and 5% by mass of polyvinylidene fluoride (PVdF) as a binder in an *N*-methylpyrrolidone solvent using a disperser. Both sides of a 21 μm-thick aluminum foil were uniformly coated with this slurry, and this aluminum foil was subsequently dried and then pressed to obtain a positive electrode.

[Production of Negative Electrode]

In 2,000 g of flake graphite having an average particle size of 35 μm, 50 g of Si fine particles having an average particle size of 0.2 μm was dispersed, and the resulting dispersion was loaded to a hybridization system (manufactured by Nara Machinery Co., Ltd.) and circulated or retained in the system for 180 seconds at a rotor rotation speed of 7,000 rpm, whereby the dispersion was treated to obtain a composite of Si and graphite particles. The thus obtained composite was mixed with coal-tar pitch, which was used as an organic compound yielding a carbonaceous substance, such that the post-firing coverage would be 7.5%, and the resulting mixture was kneaded and dispersed using a twin-screw kneader. The thus obtained dispersion was introduced to a firing furnace and fired in a nitrogen atmosphere at 1,000°C for 3 hours. The thus obtained fired product was pulverized using a hammer mill and then passed through a sieve (45 μm) to prepare a negative electrode active material. This negative electrode active material had an elemental Si content, an average particle size (d50), a tap density and a specific surface area of 2.0% by mass, 20 μm, 1.0 $g/cm^3$ and 7.2 $m^2/g$, respectively, as determined by the above-described methods.

To the thus obtained negative electrode active material, an aqueous dispersion of sodium carboxymethyl cellulose (concentration of sodium carboxymethyl cellulose = 1% by mass) and an aqueous dispersion of styrene-butadiene rubber (concentration of styrene-butadiene rubber = 50% by mass) were added as a thickening agent and a binder, respectively, and these materials were mixed using a disperser to prepare a slurry. The thus obtained slurry was uniformly coated and dried onto one side of a 10 μm-thick copper foil, and this copper foil was subsequently pressed to obtain a negative electrode. This production was carried out such that the dried negative electrode had a mass ratio (negative electrode active material: sodium carboxymethyl cellulose: styrene-butadiene rubber) of 97.5: 1.5: 1.

[Preparation of Nonaqueous Electrolyte Solutions]

To the standard electrolyte solution 1, the compounds 1 to 4 were added as additives in the respective amounts shown in Table 3 below to prepare nonaqueous electrolyte solutions. It is noted here that, in Table 3, "Content (% by mass)" indicates an amount contained in a total of 100% by mass of each nonaqueous electrolyte solution.

[Production of Nonaqueous Electrolyte Batteries]

A battery element was prepared by laminating the above-obtained positive electrode and negative electrode along with a polyethylene separator in the order of the negative electrode, the separator, and the positive electrode. This battery element was inserted into a pouch made of a laminated film obtained by coating both sides of an aluminum sheet (thickness: 40 $\mu$m) with a resin layer, with the terminals of the positive and negative electrodes protruding out of the pouch. Thereafter, each of the above-prepared nonaqueous electrolyte solutions was injected into the pouch, and the pouch was subsequently vacuum-sealed, whereby a laminate-type nonaqueous electrolyte battery was produced.

<Evaluation of Nonaqueous Electrolyte Batteries>

For each of the thus produced nonaqueous electrolyte batteries, the amount of gas generated during charged storage and the internal resistance increase rate were determined in the same manner as in Example 1-1. Table 3 below shows the ratio of the amount of gas generated during charged storage and the ratio of the internal resistance increase rate, taking the amount of gas generated during charged storage and the internal resistance increase rate in Comparative Example 3-1 as 100, respectively.

Table 3

| | Content of Compound 1 (% by mass) | Content of Compound 2 (% by mass) | Content of Compound 3 (% by mass) | Content of Compound 4 (% by mass) | Ratio of amount of gas generated during charged storage | Ratio of internal resistance increase rate |
|---|---|---|---|---|---|---|
| Example3-1 | 1.25 | 1.00 | 0.20 | - | 38 | 93 |
| Example3-2 | 1.25 | 1.00 | - | 0.20 | 33 | 98 |
| Example3-3 | 0.75 | 1.00 | 0.15 | 0.15 | 29 | 100 |
| Comparative Example3-1 | 1.65 | 1.00 | - | - | 100 | 100 |

From the results shown in Table 3, it is apparent that the effects attributed to the use of the nonaqueous electrolyte solution of the present invention can be obtained even when a composite of Si particles and graphite particles is used as the negative electrode active material.

INDUSTRIAL APPLICABILITY

The nonaqueous electrolyte solution of the present invention is useful as a nonaqueous electrolyte solution for laminate-type batteries since it can not only improve the amount of gas generated during high-temperature storage of a nonaqueous electrolyte battery but also reduce the battery resistance.

In addition, the nonaqueous electrolyte solution of the present invention and a nonaqueous electrolyte battery including the same can be used in a variety of known applications where a nonaqueous electrolyte battery is used. Specific examples of such applications include laptop computers, stylus computers, portable computers, electronic book players, mobile phones, portable fax machines, portable copiers, portable printers, headphone stereos, video cameras, liquid crystal TVs, handy cleaners, portable CD players, mini-disc players, transceivers, electronic organizers, calculators, memory cards, portable tape recorders, radios, back-up power supplies, motors, motorcycles, motor-assisted bikes, bicycles, lighting equipment, toys, gaming machines, watches, power tools, strobe lights, cameras, household backup power sources, backup power sources for commercial use, load leveling power sources, power sources for storing natural energy, and lithium ion capacitors.

**Claims**

**1.** A nonaqueous electrolyte solution, comprising:

a compound represented by the following Formula (A);
a cyclic carbonate having an unsaturated carbon-carbon bond; and

at least one compound selected from the group consisting of a compound represented by the following Formula (B) and a compound represented by the following Formula (C),
wherein
the content of the cyclic carbonate having the unsaturated carbon-carbon bond with respect to a total amount of the nonaqueous electrolyte solution is 0.01% by mass or higher and 1.5% by mass or less,
when the nonaqueous electrolyte solution comprises only one of the compounds represented by Formula (B) and the compound represented by Formula (C), the content of the compound represented by Formula (B) or (C) with respect to the total amount of the nonaqueous electrolyte solution is 0.01% by mass or higher and 0.49% by mass or less, and
when the nonaqueous electrolyte solution comprises both of the compound represented by Formula (B) and the compound represented by Formula (C), a total content of the compound represented by Formula (B) and the compound represented by Formula (C) with respect to the total amount of the nonaqueous electrolyte solution is 0.01% by mass or higher and 0.80% by mass or less:

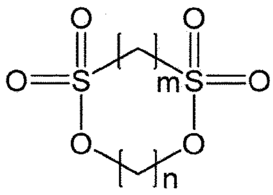
(A)

wherein, m and n each independently represent an integer of 1 to 3,

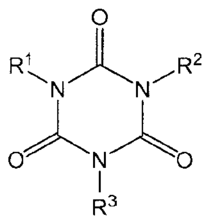
(B)

wherein, $R^1$ to $R^3$ are optionally the same or different from each other and each represent a hydrocarbon group having 1 to 10 carbon atoms which optionally has a substituent, with the proviso that at least one of $R^1$ to $R^3$ is a hydrocarbon group having an unsaturated carbon-carbon bond, and

OCN-Q-NCO (C)

wherein, Q represents a hydrocarbon group having 3 to 20 carbon atoms, and the hydrocarbon group comprises a cycloalkylene group.

2. The nonaqueous electrolyte solution according to claim 1, wherein, in Formula (B), the hydrocarbon group having an unsaturated carbon-carbon bond is an allyl group or a methallyl group.

3. The nonaqueous electrolyte solution according to claim 1 or 2, further comprising a fluorine atom-containing cyclic carbonate.

4. The nonaqueous electrolyte solution according to claim 3, wherein the content of the fluorine atom-containing cyclic carbonate is 0.01% by mass or higher and 5% by mass or less with respect to a total amount of the nonaqueous electrolyte solution.

5. The nonaqueous electrolyte solution according to any one of claims 1 to 4, further comprising at least one salt selected from the group consisting of a fluorinated salt and an oxalate salt.

6. The nonaqueous electrolyte solution according to claim 5, wherein the content of the fluorinated salt and/or the oxalate salt is 0.01% by mass or higher and 5% by mass or less with respect to a total amount of the nonaqueous electrolyte solution.

**7.** A nonaqueous electrolyte battery, comprising:

a positive electrode and a negative electrode, which are capable of absorbing and releasing metal ions; and
a nonaqueous electrolyte solution,
wherein the nonaqueous electrolyte solution is the nonaqueous electrolyte solution according to any one of claims 1 to 6.

**8.** The nonaqueous electrolyte battery according to claim 7, wherein a positive electrode active material contained in the positive electrode is a metal oxide represented by the following composition formula (1):

$$Li_{a1}Ni_{b1}CO_{c1}M_{d1}O_2 \quad (1)$$

wherein, a1, b1, c1 and d1 represent numerical values of $0.90 \leq a1 \leq 1.10$, $0.50 \leq b1 \leq 0.98$, $0.01 \leq c1 < 0.50$ and $0.01 \leq d1 < 0.50$, satisfying $b1 + c1 + d1 = 1$; and M represents at least one element selected from the group consisting of Mn, Al, Mg, Zr, Fe, Ti, and Er.

**9.** The nonaqueous electrolyte battery according to claim 7 or 8, wherein the negative electrode comprises a negative electrode active material that comprises metal particles alloyable with Li, and a graphite.

**10.** The nonaqueous electrolyte battery according to claim 9, wherein the metal particles alloyable with Li are metal particles comprising at least one metal selected from the group consisting of Si, Sn, As, Sb, Al, Zn, and W.

**11.** The nonaqueous electrolyte battery according to claim 9, wherein the metal particles alloyable with Li are composed of Si or Si metal oxide.

**12.** The nonaqueous electrolyte battery according to any one of claims 9 to 11, wherein the negative electrode active material that comprises the metal particles alloyable with Li and the graphite is a composite and/or a mixture of metal particles and graphite particles.

**13.** The nonaqueous electrolyte battery according to any one of claims 9 to 12, wherein the content of the metal particles alloyable with Li is 0.1% by mass or higher and 25% by mass or less with respect to a total amount of the negative electrode active material that comprises the metal particles alloyable with Li and the graphite.

## Patentansprüche

**1.** Eine nichtwässrige Elektrolytlösung, umfassend:

eine Verbindung, dargestellt durch die nachstehende Formel (A);
ein cyclisches Carbonat mit einer ungesättigten Kohlenstoff-Kohlenstoff-Bindung; und
mindestens eine Verbindung, ausgewählt aus der Gruppe bestehend aus einer Verbindung, dargestellt durch die nachstehende Formel (B), und einer Verbindung, dargestellt durch die nachstehende Formel (C),
wobei
der Gehalt des cyclischen Carbonats mit der ungesättigten Kohlenstoff-Kohlenstoff-Bindung, bezogen auf eine Gesamtmenge der nichtwässrigen Elektrolytlösung, 0,01 Massen-% oder mehr und 1,5 Massen-% oder weniger beträgt,
wenn die nichtwässrige Elektrolytlösung nur eine der Verbindungen, dargestellt durch Formel (B), und der Verbindung, dargestellt durch Formel (C), umfasst, der Gehalt der Verbindung, dargestellt durch Formel (B) oder (C), bezogen auf die Gesamtmenge der nichtwässrigen Elektrolytlösung, 0,01 Massen-% oder mehr und 0,49 Massen-% oder weniger beträgt und
wenn die nichtwässrige Elektrolytlösung sowohl die Verbindung, dargestellt durch Formel (B), als auch die Verbindung, dargestellt durch Formel (C), umfasst, ein Gesamtgehalt der Verbindung, dargestellt durch Formel (B), und der Verbindung, dargestellt durch Formel (C), bezogen auf die Gesamtmenge der nichtwässrigen Elektrolytlösung, 0,01 Massen-% oder mehr und 0,80 Massen-% oder weniger beträgt:

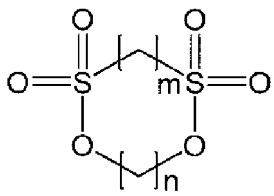

(A)

wobei m und n jeweils unabhängig eine ganze Zahl von 1 bis 3 darstellen,

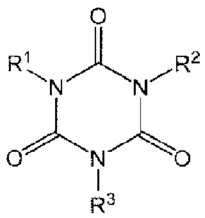

(B)

wobei $R^1$ bis $R^3$ gegebenenfalls gleich oder verschieden voneinander sind und jeweils eine Kohlenwasserstoffgruppe mit 1 bis 10 Kohlenstoffatomen darstellen, die gegebenenfalls einen Substituenten aufweist, mit der Maßgabe, dass mindestens einer von $R^1$ bis $R^3$ eine Kohlenwasserstoffgruppe mit einer ungesättigten Kohlenstoff-Kohlenstoff-Bindung ist, und

OCN-Q-NCO (C)

wobei Qeine Kohlenwasserstoffgruppe mit 3 bis 20 Kohlenstoffatomen darstellt und die Kohlenwasserstoffguppe eine Cycloalkylengruppe umfasst.

**2.** Die nichtwässrige Elektrolytlösung gemäß Anspruch 1, wobei in Formel (B) die Kohlenwasserstoffgruppe mit einer ungesättigten Kohlenstoff-Kohlenstoff-Bindung eine Allylgruppe oder eine Methallylgruppe ist.

**3.** Die nichtwässrige Elektrolytlösung gemäß Anspruch 1 oder 2, ferner umfassend ein Fluoratom enthaltendes cyclisches Carbonat.

**4.** Die nichtwässrige Elektrolytlösung gemäß Anspruch 3, wobei der Gehalt des Fluoratom enthaltenden cyclischen Carbonats 0,01 Massen-% oder mehr und 5 Massen-% oder weniger, bezogen auf eine Gesamtmenge der nichtwässrigen Elektrolytlösung, beträgt.

**5.** Die nichtwässrige Elektrolytlösung gemäß einem der Ansprüche 1 bis 4, ferner umfassend mindestens ein Salz, ausgewählt aus der Gruppe bestehend aus einem fluorierten Salz und einem Oxalatsalz.

**6.** Die nichtwässrige Elektrolytlösung gemäß Anspruch 5, wobei der Gehalt des fluorierten Salzes und/oder des Oxalatsalzes 0,01 Massen-% oder mehr und 5 Massen-% oder weniger, bezogen auf eine Gesamtmenge der nichtwässrigen Elektrolytlösung, beträgt.

**7.** Eine Batterie mit nichtwässrigem Elektrolyt, umfassend:

eine positive Elektrode und eine negative Elektrode, die Metallionen absorbieren und freisetzen können; und eine nichtwässrige Elektrolytlösung,
wobei die nichtwässrige Elektrolytlösung die nichtwässrige Elektrolytlösung gemäß einem der Ansprüche 1 bis 6 ist.

**8.** Die Batterie mit nichtwässrigem Elektrolyt gemäß Anspruch 7, wobei ein in der positiven Elektrode enthaltenes Positivelektroden-Aktivmaterial ein Metalloxid, dargestellt durch die nachstehende Zusammensetzungsformel (1), ist:

$Li_{a1}Ni_{b1}Co_{c1}M_{d1}O_2$ (1)

wobei a1, b1, c1 und d1 numerische Werte von $0,90 \leq a1 \leq 1,10$, $0,50 \leq b1 \leq 0,98$, $0,01 \leq c1 < 0,50$ und $0,01 \leq d1 < 0,50$ darstellen, die $b1 + c1 + d1 = 1$ erfüllen; und M mindestens ein Element, ausgewählt aus der Gruppe bestehend aus Mn, Al, Mg, Zr, Fe, Ti, und Er, darstellt.

**9.** Die Batterie mit nichtwässrigem Elektrolyt gemäß Anspruch 7 oder 8, wobei die negative Elektrode ein Negativelektroden-Aktivmaterial umfasst, das mit Li legierbare Metallteilchen und ein Graphit umfasst.

**10.** Die Batterie mit nichtwässrigem Elektrolyt gemäß Anspruch 9, wobei die mit Li legierbaren Metallteilchen Metallteilchen sind, die mindestens ein Metall, ausgewählt aus der Gruppe bestehend aus Si, Sn, As, Sb, Al, Zn und W, umfassen.

**11.** Die Batterie mit nichtwässrigem Elektrolyt gemäß Anspruch 9, wobei die mit Li legierbaren Metallteilchen aus Si oder Si-Metalloxid bestehen.

**12.** Die Batterie mit nichtwässrigem Elektrolyt gemäß einem der Ansprüche 9 bis 11, wobei das Negativelektroden-Aktivmaterial, das die mit Li legierbaren Metallteilchen und das Graphit umfasst, ein Verbundmaterial und/oder ein Gemisch aus Metallteilchen und Graphitteilchen ist.

**13.** Die Batterie mit nichtwässrigem Elektrolyt gemäß einem der Ansprüche 9 bis 12, wobei der Gehalt der mit Li legierbaren Metallteilchen 0,1 Massen-% oder mehr und 25 Massen-% oder weniger, bezogen auf eine Gesamtmenge des Negativelektroden-Aktivmaterials, das die mit Li legierbaren Metallteilchen und das Graphit umfasst, beträgt.

## Revendications

**1.** Solution d'électrolyte non aqueuse, comprenant :

un composé représenté par la formule (A) suivante ;
un carbonate cyclique ayant une liaison carbone-carbone insaturée ; et
au moins un composé choisi dans le groupe consistant en un composé représenté par la formule (B) suivante et un composé représenté par la formule (C) suivante,
dans laquelle
la teneur du carbonate cyclique ayant la liaison carbone-carbone insaturée par rapport à une quantité totale de la solution d'électrolyte non aqueuse est de 0,01 % en masse ou supérieure et 1,5 % en masse ou inférieure,
lorsque la solution d'électrolyte non aqueuse comprend uniquement un du composé représenté par la formule (B) et du composé représenté par la formule (C), la teneur du composé représenté par la formule (B) ou (C) par rapport à la quantité totale de la solution d'électrolyte non aqueuse est de 0,01 % en masse ou supérieure et 0,49 % en masse ou inférieure, et
lorsque la solution d'électrolyte non aqueuse comprend à la fois le composé représenté par la formule (B) et le composé représenté par la formule (C), une teneur totale du composé représenté par la formule (B) et du composé représenté par la formule (C) par rapport à la quantité totale de la solution d'électrolyte non aqueuse est de 0,01 % en masse ou supérieure et 0,80 % en masse ou inférieure :

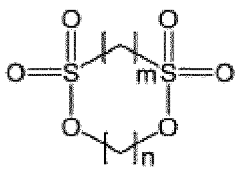

(A)

dans laquelle, m et n représentent chacun indépendamment un nombre entier de 1 à 3,

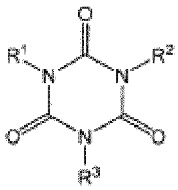

(B)

dans laquelle, $R^1$ à $R^3$ sont éventuellement identiques ou différents l'un de l'autre et chacun représente un groupe hydrocarboné ayant de 1 à 10 atomes de carbone qui présente éventuellement un substituant, à condition qu'au moins un de $R^1$ à $R^3$ soit un groupe hydrocarboné ayant une liaison carbone-carbone insaturée, et

OCN-Q-NCO (C)

dans laquelle, Q représente un groupe hydrocarboné ayant de 3 à 20 atomes de carbone, et le groupe hydrocarboné comprend un groupe cycloalkylène.

**2.** Solution d'électrolyte non aqueuse selon la revendication 1, dans laquelle, dans la formule (B), le groupe hydrocarboné ayant une liaison carbone-carbone insaturée est un groupe allyle ou un groupe méthallyle.

**3.** Solution d'électrolyte non aqueuse selon la revendication 1 ou 2, comprenant de plus un carbonate cyclique contenant un atome de fluor.

**4.** Solution d'électrolyte non aqueuse selon la revendication 3, dans laquelle la teneur du carbonate cyclique contenant un atome de fluor est de 0,01 % en masse ou supérieure et 5 % en masse ou inférieure par rapport à une quantité totale de la solution d'électrolyte non aqueuse.

**5.** Solution d'électrolyte non aqueuse selon l'une quelconque des revendications 1 à 4, comprenant de plus au moins un sel choisi dans le groupe consistant en un sel fluoré et un sel d'oxalate.

**6.** Solution d'électrolyte non aqueuse selon la revendication 5, dans laquelle la teneur du sel fluoré et/ou du sel d'oxalate est de 0,01 % en masse ou supérieure et 5 % en masse ou inférieure par rapport à une quantité totale de la solution d'électrolyte non aqueuse.

**7.** Accumulateur à électrolyte non aqueux, comprenant :

une électrode positive et une électrode négative, qui sont capables d'absorber et libérer des ions de métaux ; et une solution d'électrolyte non aqueuse,
dans lequel la solution d'électrolyte non aqueuse est la solution d'électrolyte non aqueuse selon l'une quelconque des revendications 1 à 6.

**8.** Accumulateur à électrolyte non aqueux selon la revendication 7, dans lequel une matière active d'électrode positive contenue dans l'électrode positive est un oxyde de métal représenté par la formule de composition (1) suivante :

$$Li_{a1}Ni_{b1}Co_{c1}M_{d1}O_2 \qquad (1)$$

dans laquelle, a1, b1, c1 et d1 représentent les valeurs numériques de $0{,}90<a1<1{,}10$, $0{,}50<b1<0{,}98$, $0{,}01<c1<0{,}50$ et $0{,}01<d1<0{,}50$, satisfaisant $b1 + c1 + d1 = 1$ ; et M représente au moins un élément choisi dans le groupe consistant en Mn, Al, Mg, Zr, Fe, Ti, et Er.

**9.** Accumulateur à électrolyte non aqueux selon la revendication 7 ou 8, dans lequel l'électrode négative comprend une matière active d'électrode négative qui comprend des particules de métal pouvant être alliées avec Li, et un graphite.

**10.** Accumulateur à électrolyte non aqueux selon la revendication 9, dans lequel les particules de métal pouvant être alliées avec Li sont des particules de métal comprenant au moins un métal choisi dans le groupe consistant en Si, Sn, As, Sb, Al, Zn, et W.

11. Accumulateur à électrolyte non aqueux selon la revendication 9, dans lequel les particules de métal pouvant être alliées avec Li sont constituées de Si ou d'un oxyde de Si métal.

12. Accumulateur à électrolyte non aqueux selon l'une quelconque des revendications 9 à 11, dans lequel la matière active d'électrode négative qui comprend les particules de métal pouvant être alliées avec Li et le graphite est un composite et/ou un mélange de particules de métal et de particules de graphite.

13. Accumulateur à électrolyte non aqueux selon l'une quelconque des revendications 9 à 12, dans lequel la teneur des particules de métal pouvant être alliées avec Li est de 0,1 % en masse ou supérieure et 25 % en masse ou inférieure par rapport à une quantité totale de matière active d'électrode négative qui comprend les particules de métal pouvant être alliées avec Li et le graphite.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013146819 A
- JP 2010282906 A
- JP 2011044339 A
- JP 2011048987 A
- EP 3051618 A1
- JP 2016054143 A
- US 2004043300 A1
- US 2015364794 A1